# FASCICULE DE BREVET EUROPEEN

(11) **EP 2 601 700 B1**
(45) Date de publication et mention de la délivrance du brevet: **07.09.2016**
(21) Numéro de dépôt: 11731324.7
(22) Date de dépôt: 06.07.2011
(51) Int. Cl.: H01M 8/10, C07D 251/24, C08J 5/22, C08G 73/06, B01D 71/62, B01D 67/00, B01D 71/32, B01D 71/66, B01D 71/68, B01D 71/82, H01M 8/1027, H01M 8/1032, H01M 8/1039, H01M 8/1018, H01M 8/103

(54) **MONOMÈRE TRIAZINE SOUFRÉ UTILISABLE POUR LA SYNTHÈSE D'UNE MEMBRANE POLYMÈRE POUR PILE À COMBUSTIBLE**
TRIAZINMONOMERE UND DEREN VERWENDUNG ZUR HERSTELLUNG VON POLYMERMEMBRANEN FÜR BRENNSTOFFZELLEN
TRIAZINE MONOMERS AND THEIR USE IN THE PREPARATION OF POLYMERIC MEMBRANES IN FUEL CELLS

(30) Priorité: 04.08.2010 FR 1056438
(43) Date de publication de la demande: 12.06.2013
(73) Titulaire: Compagnie Générale des Etablissements Michelin, 63000 Clermont-Ferrand (FR); Michelin Recherche et Technique S.A., 1763 Granges-Paccot (CH)
(72) Inventeur: FEDURCO, Milan, F-63040 CLERMONT-FERRAND Cedex 9 (FR)
(74) Mandataire: Ribière, Joel
(86) Numéro de dépôt international: PCT/EP2011/061424
(87) Numéro de publication internationale: WO 2012/016778

(56) Documents cités:
- US-A1- 2005 221 135

## Description

### I. DOMAINE DE L'INVENTION

La présente invention est relative aux monomères utilisables pour la synthèse de polymères destinés notamment, sous forme sulfonés, à constituer un électrolyte solide ou membrane dans une pile à combustible.

Elle est plus particulièrement relative aux monomères ci-dessus du type aromatiques et comportant une unité structurelle de base à noyau triazine.

### II. ETAT DE LA TECHNIQUE

L'intérêt récent pour les piles à combustible vient de leur capacité à convertir l'énergie chimique en électricité avec un rendement relativement élevé et une émission basse de polluants environnementaux. Aujourd'hui, l'utilisation de tels dispositifs électrochimiques s'étend de l'industrie automobile aux ordinateurs portatifs, aux téléphones cellulaires, à la génération stationnaire d'énergie électrique, ainsi qu'à d'autres applications comprenant l'exploration de la mer et de l'espace.

On rappellera tout d'abord qu'une pile à combustible est un générateur d'énergie électrochimique dans lequel est entretenue sous contrôle une réaction chimique entre l'hydrogène et l'oxygène qui va produire de l'eau (réaction inverse de l'électrolyse). Elle produit de l'énergie électrique et de la chaleur. L'électrolyte y est constitué typiquement d'une membrane polymère PEM (abréviation pour *"Polymer Electrolyte Membrane"*) conductrice de protons et apte à séparer les espèces réactives, constituée de deux nanophases bien distinctes : d'une part une partie hydrophobe assurant l'intégrité mécanique, étanche à l'eau et aux gaz (H₂ et O₂), d'autre part une partie sulfonée constituée de canaux hydrophiles étroits permettant le passage des protons et assurant donc la conductivité ionique de la pile. Cette membrane polymère est disposée entre l'anode et la cathode de la pile, un tel assemblage étant communément appelé "MEA" (pour *Membrane Electrode Assembly*).

De tels piles à combustible, assemblages MEA ainsi que leurs principes généraux de fonctionnement sont bien connus, ils ont été décrits dans un très grand nombre de documents ; à titre d'exemples, on peut citer l'article général intitulé *"*Fatnctional fluoropolymers for fUel cell membranes" de Renaud Souzy & Bruno Ameduri, Prog. Polymer Sci. 30 (2005), 644-687, ainsi que les demandes de brevet WO 2005/006472, WO 2006/012953, WO 2006/012954, WO 2006/100029, WO 2008/125174.

Un matériau polymérique bon candidat pour une pile à combustible PEM doit satisfaire à de très hautes exigences en ce qui concerne ses propriétés mécaniques, physiques et chimiques. Idéalement, on attend de l'assemblage MEA qu'il puisse fonctionner pendant des milliers d'heures à des températures relativement élevées (60 à 100°C dans le cas de piles PEM, jusqu'à 160°C dans le cas de piles au méthanol dites DMFC) tout en étant exposé à une humidité particulièrement élevée et des valeurs de pH acide proches de zéro. La plupart des polymères connus subissent une décomposition sous de telles conditions, qu'ils soient de type aliphatiques comme aromatiques.

Des copolymères aliphatiques dérivés d'acide perfluorosulfonique, commercialisés par exemple sous le nom de Nafion^{®} ou Flemion^{®}, ont été employés intensivement comme membranes conductrices dans des piles à combustible du type hydrogène/ air, hydrogène/ oxygène ou méthanol/ air.

Malgré une très bonne conductivité ionique et une haute stabilité chimique, l'emploi de polymères du type Nafion^{®} n'est tout d'abord pas adapté à une utilisation dans des piles à combustible du type méthanol, cela en raison d'une performance réduite pour les températures d'utilisation les plus élevées, due à une augmentation importante de perméabilité de la membrane vis-à-vis du méthanol.

Un autre inconvénient connu des polymères du type Nafion^{®}, en fonctionnement dans la pile, est leur stabilité chimique relativement limitée. En effet, les polymères perfluorés sont connus pour absorber des quantités importantes d'eau responsables de gonflements et de changements dimensionnels répétés de la membrane : des cycles répétés de séchage et d'humidification, lors des arrêts et démarrages successifs de la pile à combustible, entraînent une perméabilité accrue aux gaz (H₂ et O₂) ; cette perméabilité accrue est responsable de la formation d'eau oxygénée et de radicaux libres (OH), autant de mécanismes qui peuvent conduire à une dégradation rapide de la membrane et à une fin de vie prématurée de la pile à combustible. Pour limiter ces changements dimensionnels et améliorer ainsi l'endurance des membranes, il a notamment été proposé d'ajouter, à titre de polymère de renforcement, un second polymère fluoré, notamment un PTFE (polytétrafluoroéthylène) du type microporeux expansé (ou "ePTFE"), et de constituer ainsi des membranes composites plus endurantes (voir par exemple US 6 495 209).

Enfin, un autre inconvénient majeur des polymères du type Nafion^{®} est leur coût de synthèse, sans parler d'une chimie de base qui ne répond plus aujourd'hui aux exigences les plus récentes en termes d'environnement, de règles d'hygiène et sécurité.

Aussi, de nombreuses recherches ont été conduites dans le passé pour tenter de réduire le coût des membranes PEM.

Il a été proposé notamment de remplacer les polymères aliphatiques ci-dessus par des polymères aromatiques, de coût inférieur et qui ont par ailleurs comme avantage de présenter une perméabilité aux gaz (H₂ et O₂) qui est réduite.

Des exemples de tels polymères sont par exemple des poly(arylène-éther-sulfone), commercialisés notamment sous les dénominations "Udel", "Radel" ou encore des poly (éther-éther-cétone) commercialisés par exemple sous dénomination "PEEK". Les polymères aromatiques ci-dessus, une fois sulfonés, ne permettent pas encore aujourd'hui d'atteindre le compromis de performances et de coût offert avec les polymères aliphatiques fluorés du type Nafion^{®}. En outre, ces polymères aromatiques se mélangent généralement mal avec un polymère type ePTFE et les membranes qui en sont issues ne peuvent donc être renforcées facilement par un polymère ePTFE, un tel renforcement exigeant un traitement de surface préalable du polymère ePTFE par plasma ou par voie chimique dans des milieux chimiques très agressifs (voir par exemple article intitulé *"*Challenging reinforced composite polymer electrolyte membranes based on disulfonated poly(arylene-ether-sulfone)-impregnated expanded PTFE for fuel cell applications", Xiaobing Zhu et al, J. Mat. Chem., 2007, 386-397).

D'autres exemples de polymères du type aromatiques ont été décrits plus récemment dans les documents brevet US2005/0221135 et US 7037614. Il s'agit de polymères triazine sulfonés dont les monomères sont connectés par des ponts éther (-O-) Les synthèses décrites dans ces documents sont complexes, coûteuses et difficiles à reproduire. On a constaté en outre que leur stabilité, chimique et dimensionnelle, est insuffisante même après un traitement final de réticulation des membranes, traitement qui nécessite par ailleurs une autre chimie complexe et coûteuse.

### III. BREVE DESCRIPTION DE L'INVENTION

Au cours de leurs recherches, les Demanderesses ont trouvé un monomère aromatique nouveau, plus précisément un monomère à noyau triazine spécifique, qui est utilisable pour la synthèse d'une membrane polymère permettant de pallier, au moins en partie, les inconvénients précités.

Ce monomère triazine de l'invention est un monomère triazine soufré répondant à la formule : 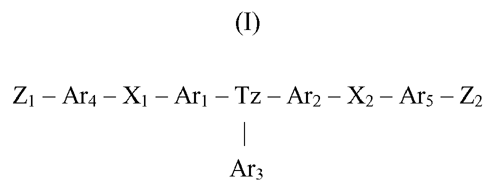 dans laquelle:
- le symbole Tz représente le noyau triazine-1,3,5 ;
- les symboles X₁ et X₂, identiques ou différents, représentent S, SO ou SO₂ ;
- les symboles Ar₁, Ar₂, Ar₄ et Ar₅, identiques ou différents, représentent un groupe phénylène, substitué ou non substitué ;
- le symbole Ar₃ représente un groupe phényle, substitué ou non substitué ;
- les symboles Z₁ et Z₂, identiques ou différents, sont choisis dans le groupe constitué par les halogènes, hydroxyle, alcoxyles, thiol, carboxyles, carboxylates, amino, sulfonamido, chlorure d'acyle, chlorure de sulfonyle, fluorure de sulfonyle, et isocyanate.

Grâce à ce monomère triazine conforme à l'invention, il est possible de préparer un polymère triazine qui, comparé aux polymères triazine de l'art antérieur précédemment décrits, possède une stabilité chimique et une résistance à l'oxydation nettement améliorées. Il permet de fabriquer des membranes PEM qui de manière inattendue, comparées à des membranes commerciales du type Nafion^{®} développées depuis longtemps, présentent une stabilité chimique et dimensionnelle et une conductivité ionique au moins équivalentes, sinon supérieures. Enfin, ce qui n'est pas son moindre avantage, le polymère triazine issu du monomère de l'invention peut être rendu compatible avec un polymère microporeux ePTFE pour un renforcement optimal de la membrane, sans nécessiter les traitements de surface qui ont été évoqués supra.

L'invention concerne également un procédé de synthèse d'un polymère triazine par polycondensation d'au moins un monomère triazine conforme à l'invention.

L'invention concerne également l'utilisation d'un monomère triazine conforme à l'invention pour la fabrication d'une membrane de polymère utilisable dans une pile à combustible du type PEM.

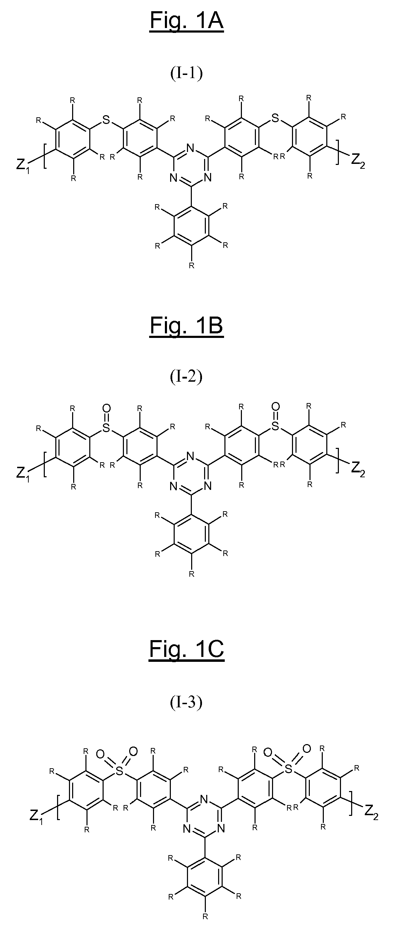

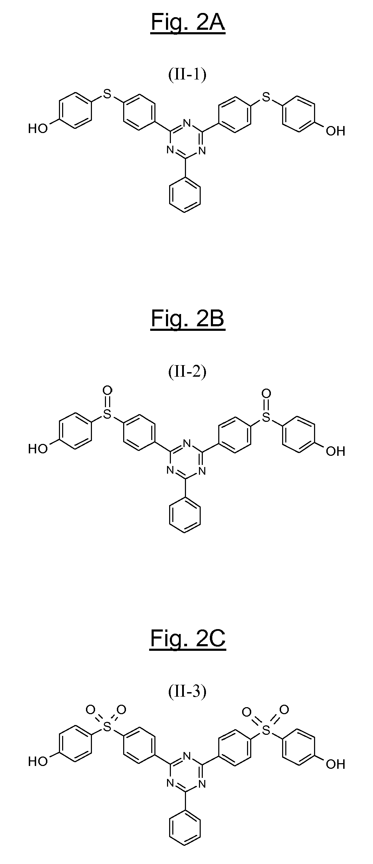

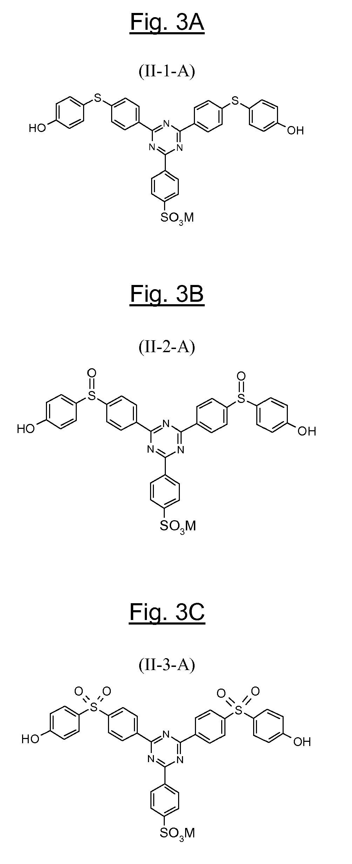

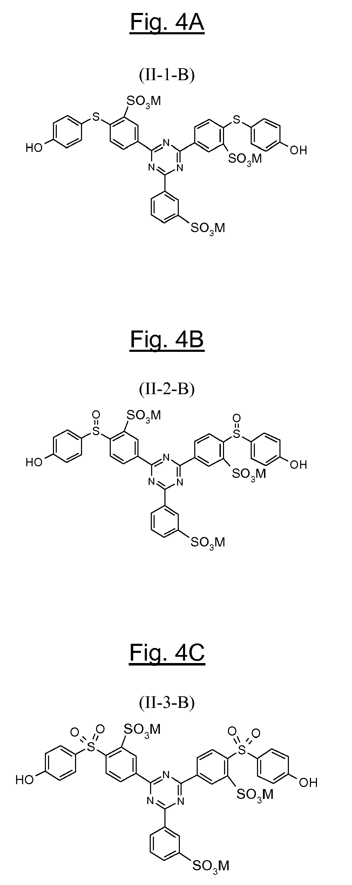

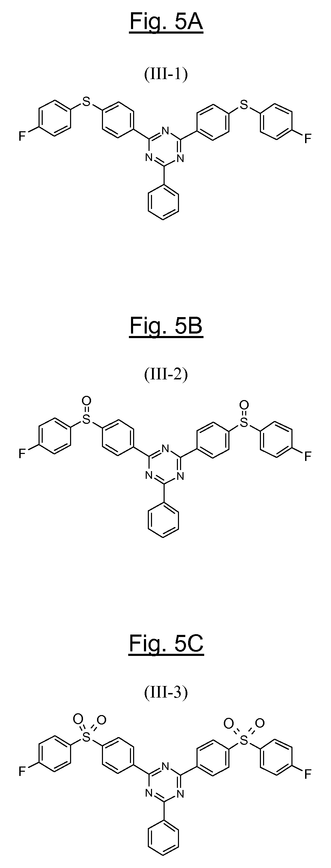

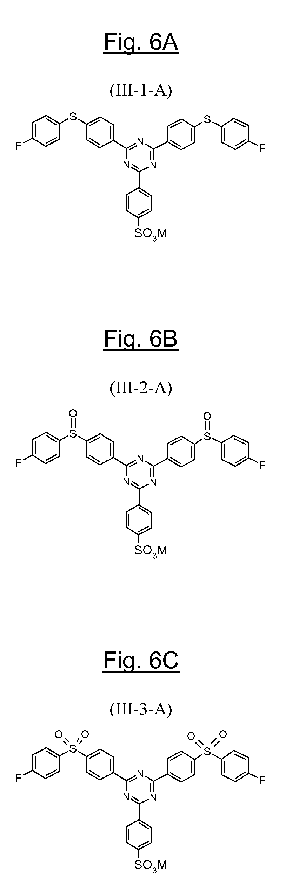

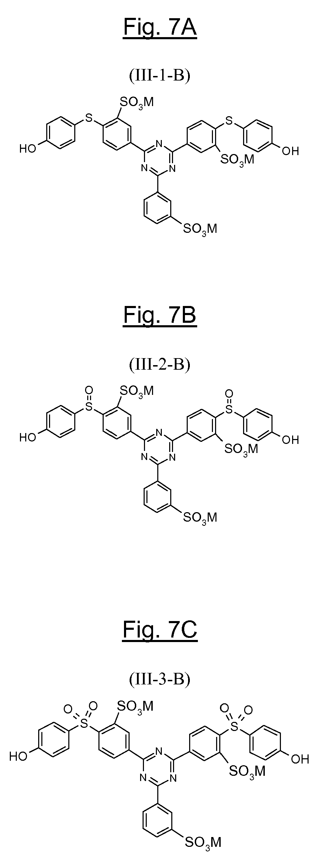

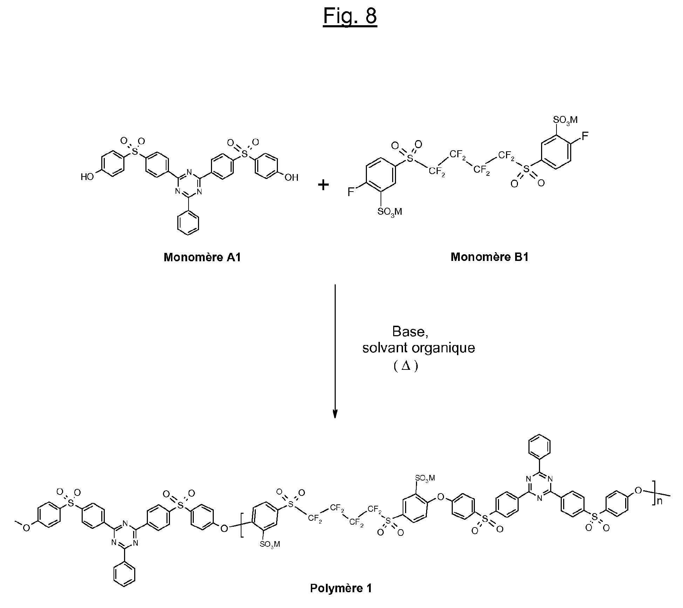

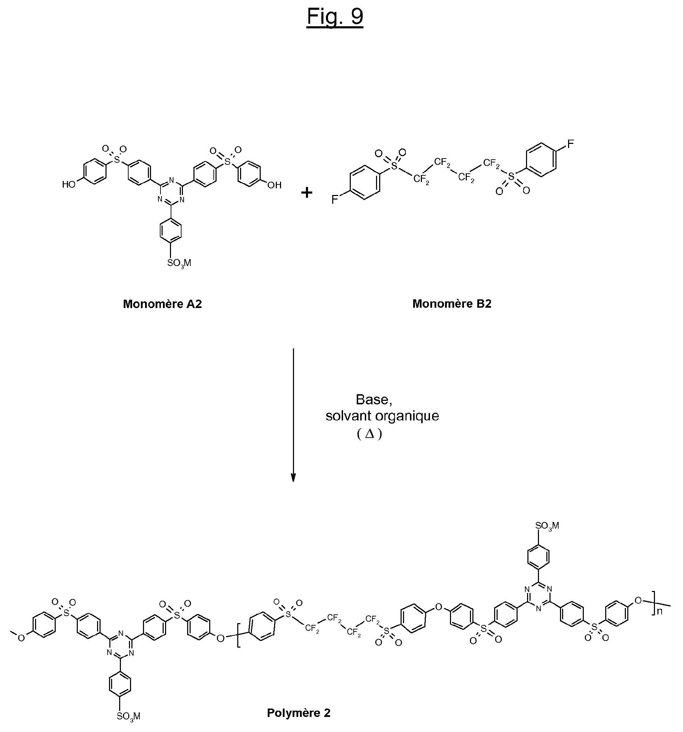

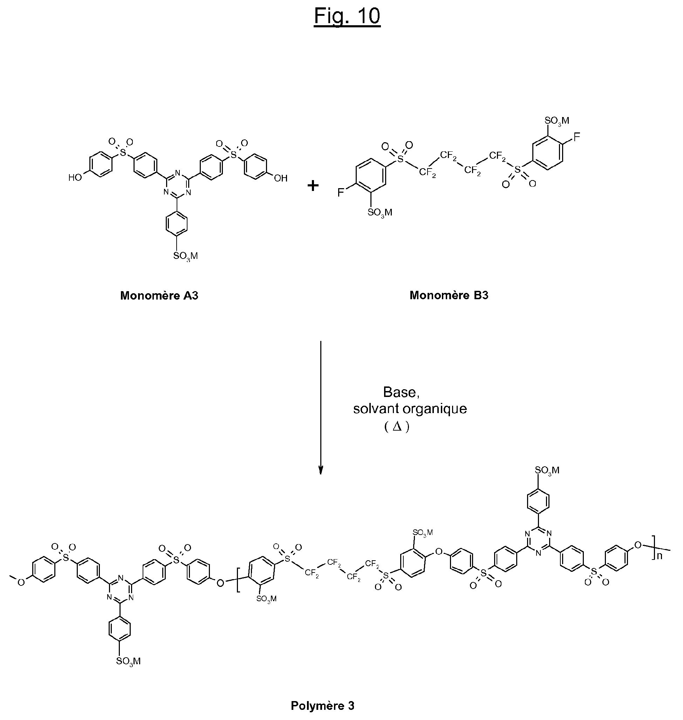

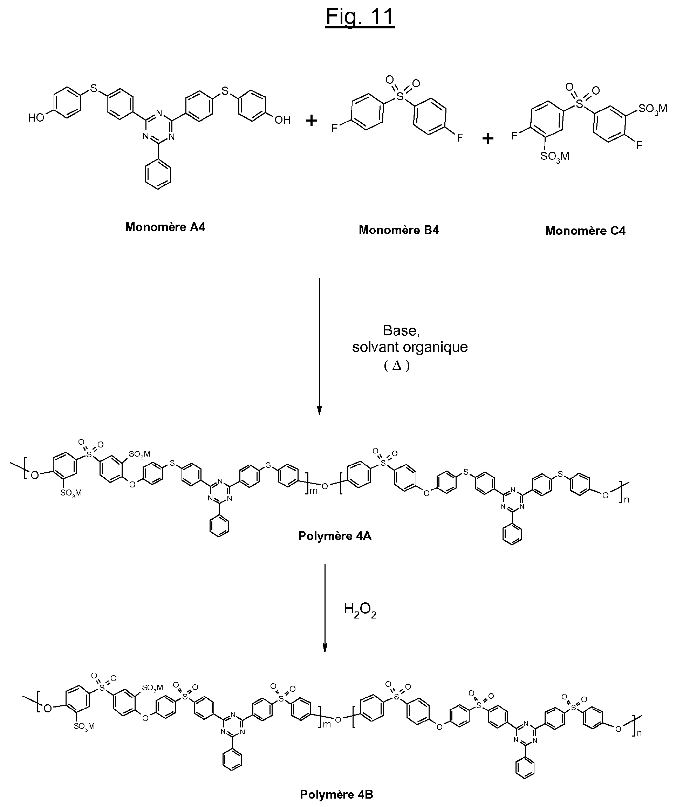

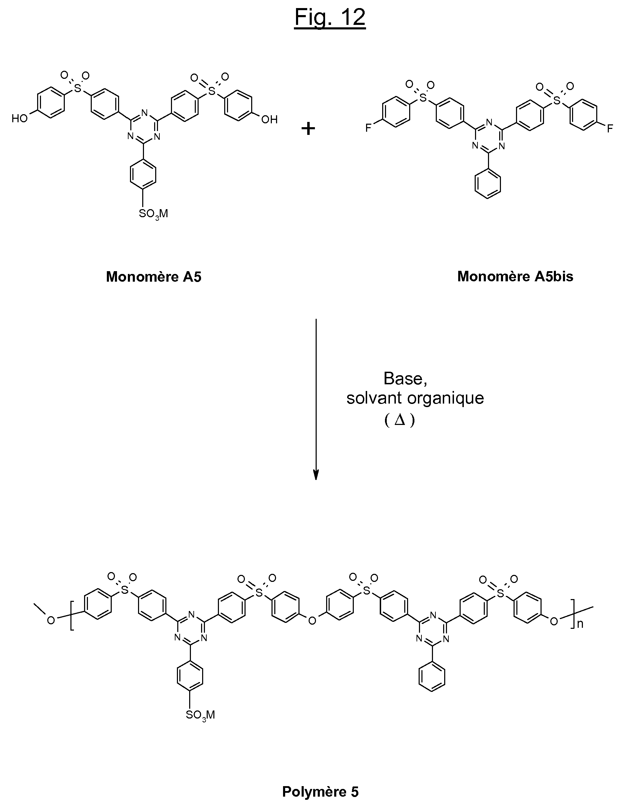

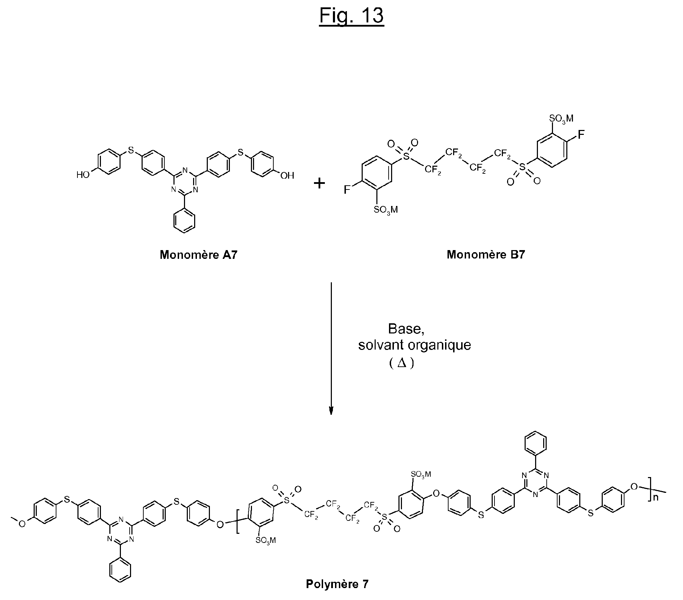

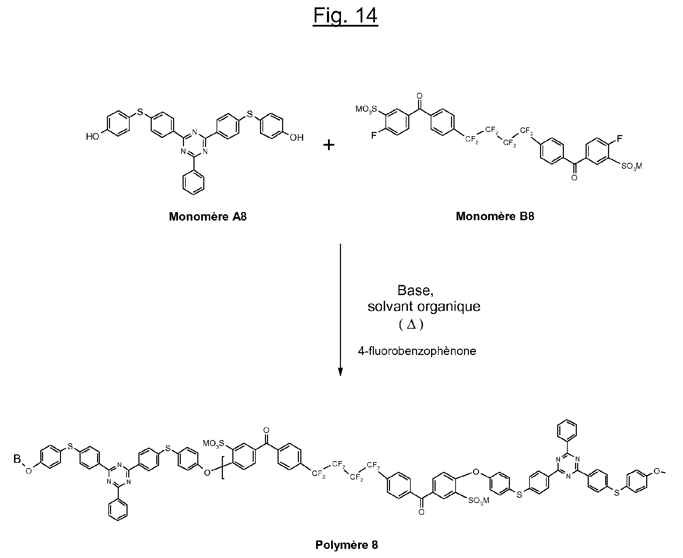

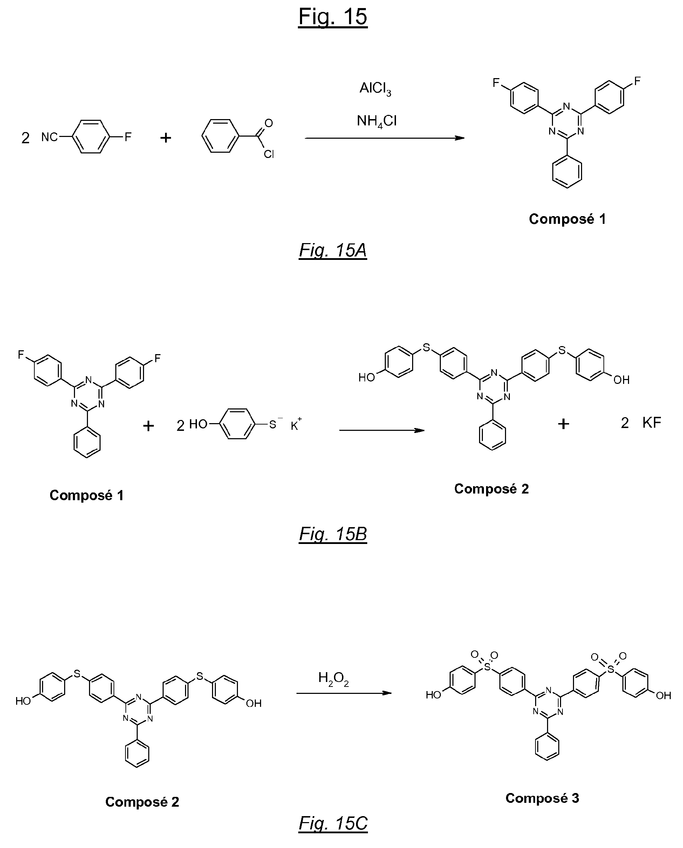

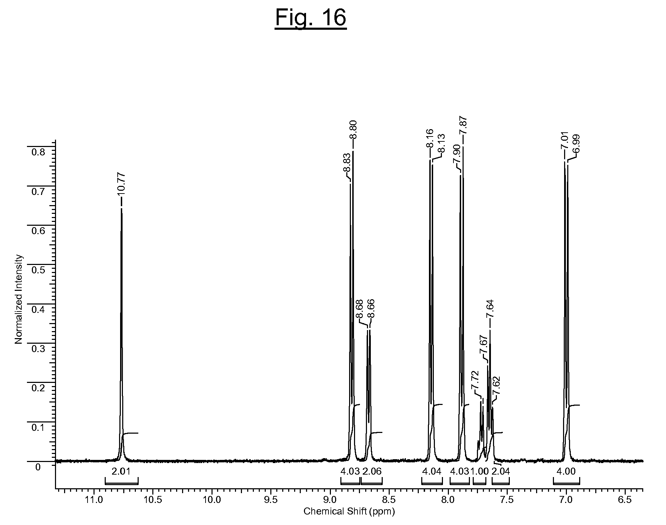

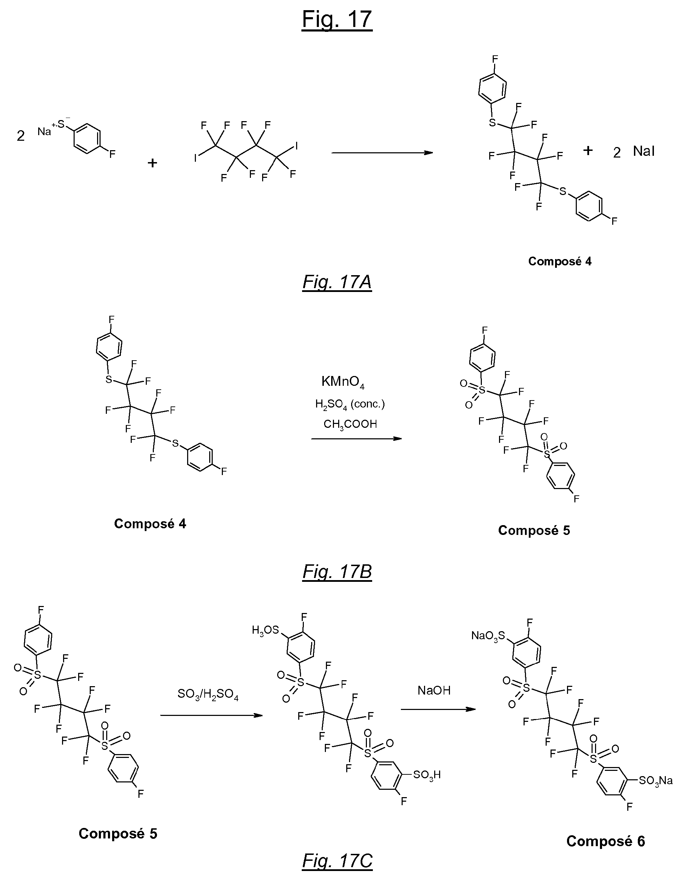

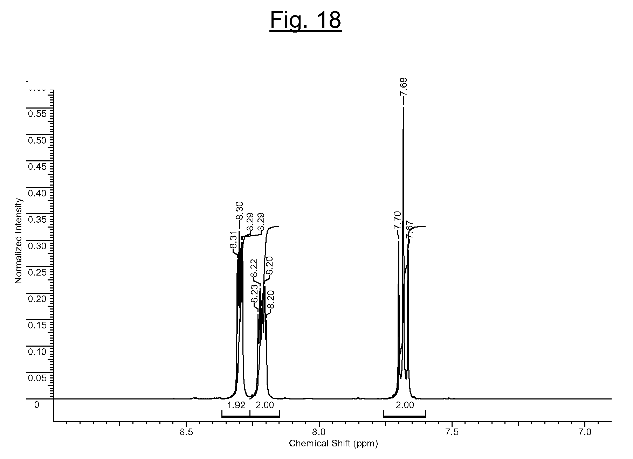

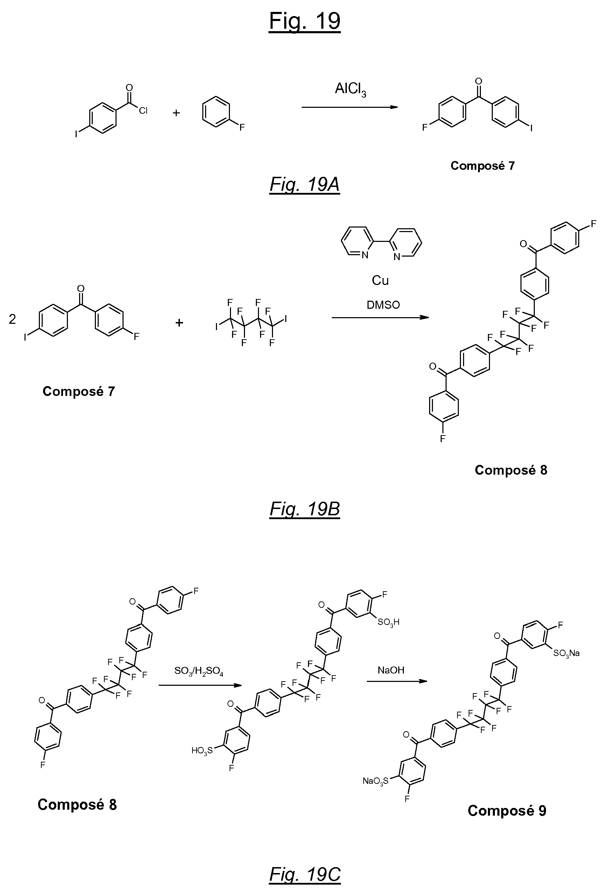

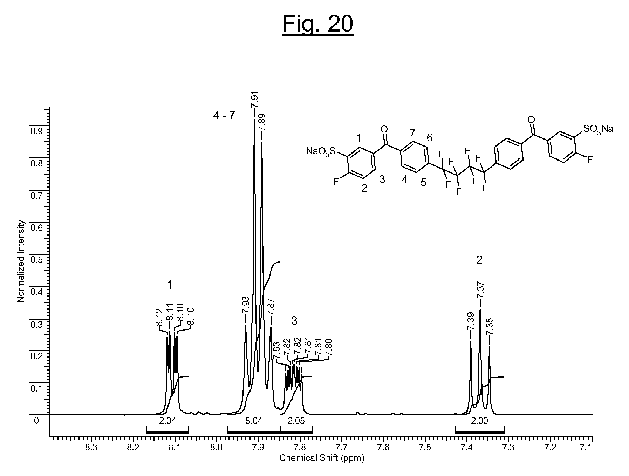

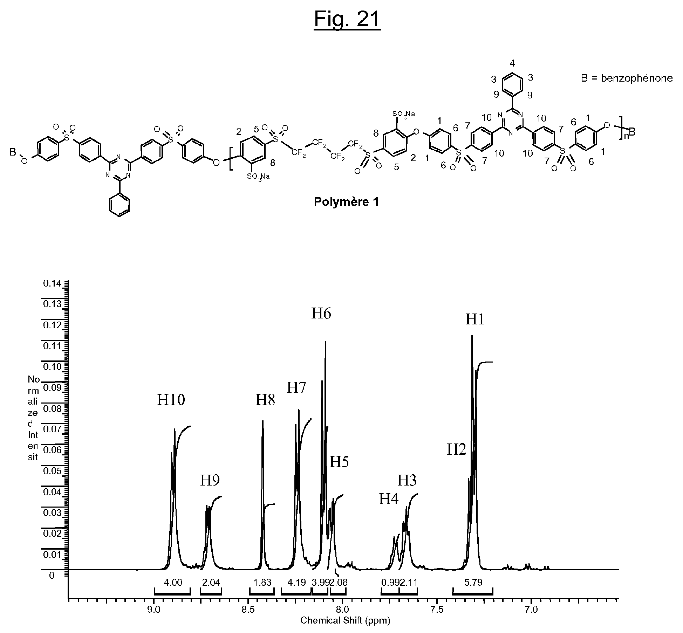

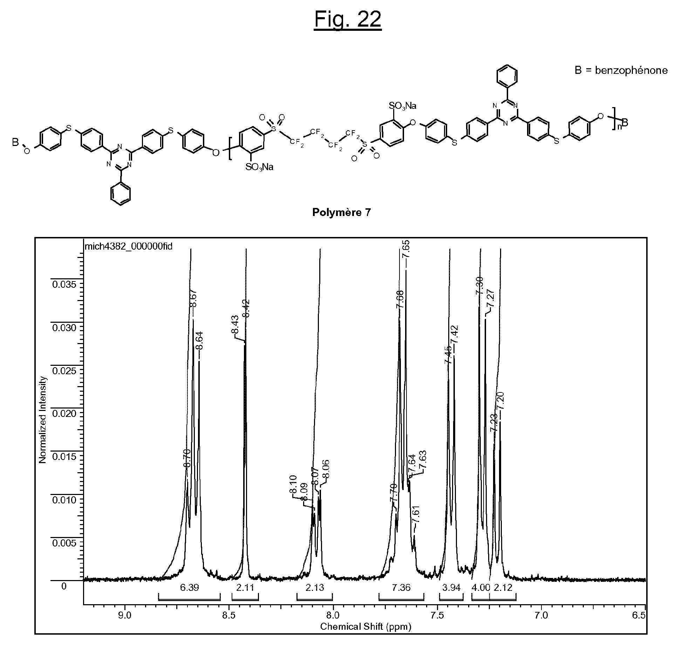

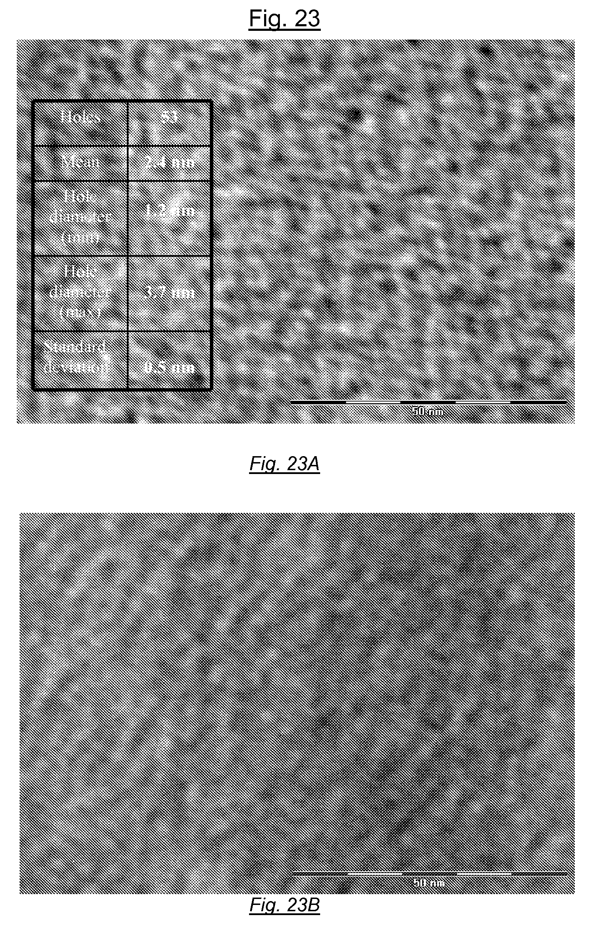

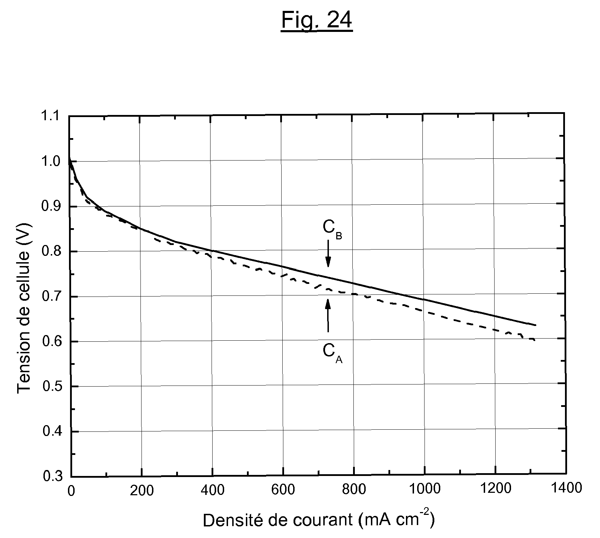

L'invention ainsi que ses avantages seront aisément compris à la lumière de la description détaillée et des exemples de réalisation qui suivent, ainsi que des figures relatives à ces exemples qui représentent ou schématisent :
- des exemples de monomères conformes à l'invention de formule (I), de formules particulières respectives (I-1), (1-2) et (1-3) (Fig. 1A, 1B et 1C) ;
- des exemples de monomères conformes à l'invention de formule (II), de formules particulières respectives (II-1), (II-2) et (II-3) (Fig. 2A, 2B et 2C) ;
- des exemples de monomères conformes à l'invention de formule (II), de formules particulières respectives (II-1-A), (II-2-A) et (II-3-A) (Fig. 3A, 3B et 3C) ;
- des exemples de monomères conformes à l'invention de formule (II), de formules particulières respectives (II-1-B), (II-2-B) et (II-3-B) (Fig. 4A, 4B et 4C) ;
- des exemples de monomères conformes à l'invention de formule (III), de formules particulières respectives (III-1), (III-2) et (III-3) (Fig. 5A, 5B et 5C) ;
- des exemples de monomères conformes à l'invention de formule (III), de formules particulières respectives (III-1-A), (III-2-A) et (III-3-A) (Fig. 6A, 6B et 6C) ;
- des exemples de monomères conformes à l'invention de formule (III), de formules particulières respectives (III-1-B), (III-2-B) et (III-3-B) (Fig. 7A, 7B et 7C) ;
- un exemple de polymère triazine (Polymère 1) ainsi qu'un schéma de synthèse possible de ce polymère par polycondensation d'un monomère A1 conforme à l'invention avec un second monomère B1 non conforme à l'invention (Fig. 8) ;
- un autre exemple de polymère triazine (Polymère 2) ainsi qu'un schéma de synthèse possible de ce polymère par polycondensation d'un monomère A2 conforme à l'invention avec un second monomère B2 non conforme à l'invention (Fig. 9) ;
- un autre exemple de polymère triazine (Polymère 3) ainsi qu'un schéma de synthèse possible de ce polymère par polycondensation d'un monomère A3 conforme à l'invention avec un second monomère B3 non conforme à l'invention (Fig. 10) ;
- deux autres exemples de polymères triazine (Polymère 4A et Polymère 4B) ainsi qu'un schéma de synthèse possible de ces polymères par polycondensation d'un monomère A4 conforme à l'invention avec deux autres monomères B4 et C4 non conformes à l'invention (Fig. 11) ;
- un autre exemple de polymère triazine (Polymère 5) ainsi qu'un schéma de synthèse possible de ce polymère par polycondensation d'un monomère A5 conforme à l'invention avec un second monomère A5bis conforme à l'invention (Fig. 12) ;
- un autre exemple de polymère triazine (Polymère 7) ainsi qu'un schéma de synthèse possible de ce polymère par polycondensation d'un monomère A7 conforme à l'invention avec un second monomère B7 non conforme à l'invention (Fig. 13) ;
- un autre exemple de polymère triazine (Polymère 8) ainsi qu'un schéma de synthèse possible de ce polymère par polycondensation d'un monomère A8 conforme à l'invention avec un second monomère B8 non conforme à l'invention (Fig. 14) ;
- un schéma de synthèse possible, en trois étapes successives, du monomère A1 (ou Composé 3) conforme à l'invention de la Fig. 8 (Fig. 15) ;
- le spectre RMN ¹H (360 MHz) du Composé 3 (monomère A1) dissous dans DMSO-*d₆* (Fig. 16) ;
- un schéma de synthèse possible, en trois étapes successives, du monomère B1 (ou Composé 6 - M représentant ici Na⁺) non conforme à l'invention de la Fig. 8 (Fig. 17) ;
- le spectre RMN ¹H (500 MHz) du Composé 6 (monomère B1) dissous dans DMSO-*d₆* (Fig. 18) ;
- un schéma de synthèse possible, en trois étapes successives, du monomère B8 (ou Composé 9 - M représentant ici Na⁺) non conforme à l'invention de la Fig. 14 (Fig. 19) ;
- le spectre RMN ¹H (500 MHz) du Composé 9 (monomère B8) dissous dans DMSO-*d₆* (Fig. 20) ;
- la formule du Polymère 1 sous forme sulfoné et bloqué benzophénone, ainsi que son spectre RMN ¹H (500 MHz), dissous dans DMSO-*d₆* (Fig. 21) ;
- la formule du Polymère 7 sous forme sulfoné et bloqué benzophénone, ainsi que son spectre RMN ¹H (500 MHz), dissous dans DMSO-*d₆* (Fig. 22) ;
- des clichés de microscopie électronique enregistrés respectivement sur une coupe horizontale (Fig. 23A) et une coupe transversale (Fig. 23B) d'une membrane PEM constituée du Polymère 1 (Fig. 23) ;
- les courbes de polarisation comparées d'une pile à combustible PEM utilisant la membrane issue du Polymère 1 (courbe C_{A}) et une membrane commerciale (courbe C_{B}) (Fig. 24).

### IV. DESCRIPTION DETAILLEE DE L'INVENTION

Le monomère triazine de l'invention a donc pour caractéristique essentielle de répondre à la formule (I) : 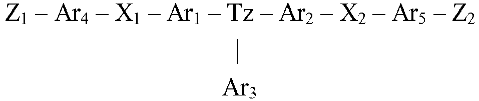 dans laquelle:
- le symbole Tz représente le noyau triazine-1,3,5 ;
- les symboles X₁ et X₂, identiques ou différents, représentent S, SO ou SO₂ ;
- les symboles Ar₁, Ar₂, Ar₄ et Ar₅, identiques ou différents, représentent un groupe phénylène, substitué ou non substitué ;
- le symbole Ar₃ représente un groupe phényle, substitué ou non substitué ;
- les symboles Z₁ et Z₂, identiques ou différents, sont choisis dans le groupe constitué par les halogènes, hydroxyle, alcoxyles, thiol, carboxyles, carboxylates, amino, sulfonamido, chlorure d'acyle, chlorure de sulfonyle, fluorure de sulfonyle, et isocyanate.

On rappellera ici que la 1,3,5-triazine (aussi dénommée "triazine s") a pour formule :

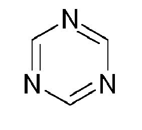

La triphényl-1,3,5-triazine est donc représentée dans la formule (I) par :

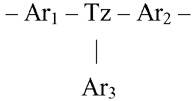

Autrement dit, le monomère triazine de l'invention de formule (I) a donc pour formule développée (le symbole R représentant ici l'hydrogène ou un substituant de l'hydrogène) :

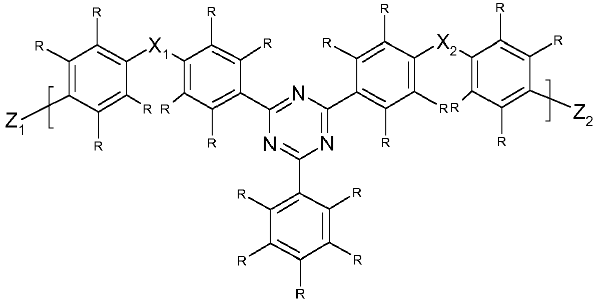

En d'autres termes, dans le cas préférentiel où X₁ et X₂ sont identiques, le monomère triazine de l'invention de formule (I) répond à l'une des trois formules 1-1, 1-2 et 1-3 représentées respectivement aux figures 1A, 1B et 1C annexées.

Comme indiqué précédemment, les groupes phényle ou phénylène Ar₁, Ar₂, Ar₃, Ar₄ et Ar₅ peuvent être substitués ou non substitués. Lorsqu'ils sont substitués, l'invention s'applique notamment aux cas où un seul groupe phényle ou phénylène par monomère triazine de formule (I) est substitué comme aux cas où plusieurs groupes phényle ou phénylène par - 8 - monomère triazine sont substitués, un seul substituant ou plusieurs substituants, identiques ou différents, pouvant être présents sur le ou les même(s) groupe(s) phényle ou phénylène.

A titre d'exemples de substituants possibles des noyaux aromatiques (c'est-à-dire plus exactement des atomes d'hydrogène de ces groupes phényle ou phénylène), on peut citer notamment les substituants suivants :
∘ - F ; - Cl ; - Br ; - CN ; - CF₃ ; - NO₂ ; - N(CH₃)₂ ;
o - COOH ; - COOM ; - PO₃H ; - PO₃M ; -SO₃H ; - SO₃M (le symbole M représentant un cation de métal alcalin, préférentiellement Na⁺ ou K⁺);
o les radicaux hydroxyle, alkyles, cycloalkyles, perfluoroalkyles, sulfoalkyles, sulfoaryles, aryles, alkylcarbonyles, arylcarbonyles, alkoxyles, aryloxyles.

Ces substituants possibles sont préférentiellement choisis dans le groupe constitué par les substituants - F, - CN, - CF₃, - PO₃H , - PO₃M , - SO₃H , - SO₃M et les mélanges de ces substituants.

Z₁ et Z₂, identiques ou différents, sont choisis dans le groupe constitué par les halogènes (tels que F, Cl, Br, I), hydroxyle (OH), alkoxyles (OR), thiol (SH), carboxyles (COOH), carboxylates (COOR), thiol (SH), amino (NH₂), sulfonamido (SO₂-NH₂), chlorure d'acyle (CO-Cl), chlorure de sulfonyle (SO₂-Cl), fluorure de sulfonyle (SO₂-F), et isocyanate (NCO):

Plus préférentiellement, Z₁ et Z₂, identiques ou différents, sont choisis dans le groupe constitué par les halogènes (tels que F, Cl, Br, I), hydroxyle (OH), et thiol (SH).

Ainsi, selon un mode de réalisation particulier et préférentiel, Z₁ et Z₂ correspondent au groupe hydroxyle. Dans le cas préférentiel où X₁ et X₂ sont identiques, le monomère triazine de l'invention de formule (I) est donc le 2,4-[(4-hydroxy-phénylsulfanyl phényl)]-6-(phényl)-[1,3,5]-triazine, 2,4-[(4-hydroxy-phénylsulfoxy-phényl)]-6-(phényl)-[1,3,5]-triazine ou 2,4-[(4-hydroxy-phénylsulfonyl phényl)]-6-(phényl)-[1,3,5]-triazine, répondant respectivement aux trois formules II-1, II-2 et II-3 représentées aux figures 2A, 2B et 2C annexées, dans lesquelles les groupes phénylène ou phényle peuvent être substitués ou non substitués.

Les figures 3A, 3B et 3C annexées représentent des exemples plus spécifiques de monomères triazine conformes à l'invention, sous forme sulfonés, de formules respectives II-1-A, II-2-A et II-3-A, dans lesquelles X₁ et X₂ sont identiques, Z₁ et Z₂ correspondent au groupe hydroxyle, et un seul des groupes phénylène ou phényle (ici, à titre d'exemple, le groupe phényle pendant Ar₃) est substitué par un groupe sulfonate SO₃M (M représentant un cation de métal alcalin, préférentiellement Na⁺ ou K⁺) ; il s'agit donc ici plus précisément, et respectivement, des sels de métal alcalin de 2,4-[(4-hydroxy-phénylsulfanyl phényl)]-6-(p-sulfonatophényl)-[1,3,5]-triazine, de 2,4-[(4-hydroxy-phénylsulfoxy-phényl)]-6-(p-sulfonatophényl)-[1,3,5]-triazine et de 2,4-[(4-hydroxy-phénylsulfonyl phényl)]-6-(p-sulfonatophényl)-[1,3,5]-triazine.

Les figures 4A, 4B et 4C annexées représentent d'autres exemples plus spécifiques de monomères triazine conformes à l'invention, sous forme sulfonés, de formules respectives II-1-B, II-2-B et II-3-B, dans lesquelles X₁ et X₂ sont identiques, Z₁ et Z₂ correspondent au groupe hydroxyle, et trois des groupes phénylène ou phényle (ici, à titre d'exemple, les groupes Ar₁, Ar₂ et Ar₃) sont substitués par un groupe sulfonate SO₃M (M représentant un cation de métal alcalin, préférentiellement Na⁺ ou K⁺) ; il s'agit donc ici plus précisément, et respectivement, des sels de métal alcalin de 2,4-bis-{(4-hydroxy-phénylsulfanyl phényl)-(3-sulfonatophényl)-6-(m-sulfonatophényl)}-[1,3,5]-triazine, de 2,4-bis-{(4-hydroxy-phénylsulfoxy phényl)-(3-sulfonatophényl)-6-(m-sulfonatophényl)}-[1,3,5]-triazine et de 2,4-bis-{(4-hydroxy-phénylsulfonyl-phényl)-(3-sulfonatophényl)-6-(m-sulfonatophényl)}-[1,3,5]-triazine.

Selon un autre mode de réalisation particulier et préférentiel, Z₁ et Z₂, identiques ou différents, correspondent à un halogène, choisi en particulier dans le groupe constitué par fluor, chlore, brome, plus particulièrement encore parmi fluor, et chlore.

Ainsi, selon un exemple de réalisation particulièrement préférentiel, Z₁ et Z₂ correspondent à l'halogène fluor, c'est-à-dire que le monomère triazine de l'invention répond alors à la formule :

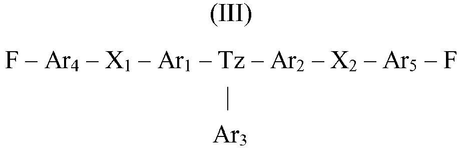

Ainsi, dans le cas plus préférentiel où X₁ et X₂ sont identiques, le monomère triazine de l'invention de formule (I) est donc le 2,4-[(4-fluoro-phénylsulfanyl phényl)]-6-(phényl)-[1,3,5]-triazine, 2,4-[(4-fluoro-phénylsulfoxy-phényl)]-6-(phényl)-[1,3,5]-triazine ou 2,4-[(4-fluoro-phénylsulfonyl-phényl)]-6-(phényl)-[1,3,5]-triazine, répondant respectivement aux formules III-1, III-2 et III-3 représentées aux figures 5A, 5B et 5C annexées, dans lesquelles les groupes phénylène ou phényle peuvent être substitués ou non substitués.

Les figures 6A, 6B et 6C annexées représentent des exemples plus spécifiques de monomères triazine conformes à l'invention, sous forme sulfonés, de formules respectives III-1-A, III-2-A et III-3-A, dans lesquelles X₁ et X₂ sont identiques, Z₁ et Z₂ correspondent au fluor, et un seul des groupes phényle (ici, à titre d'exemple, le groupe phényle pendant Ar₃) est substitué par un groupe sulfonate SO₃M (M représentant un cation de métal alcalin, préférentiellement Na⁺ ou K⁺) ; il s'agit donc ici plus précisément, et respectivement, des sels de métal alcalin de 2,4-[(4-fluoro-phénylsulfanyl phényl)]-6-(p-sulfonatophényl)-[1,3,5]-triazine, de 2,4-[(4-fluoro-phénylsulfoxy-phényl)]-6-(p-sulfonatophényl)-[1,3,5]-triazine, et de 2,4-[(4-fluoro-phénylsulfonyl phényl)]-6-(p-sulfonatophényl)-[1,3,5]-triazine.

Les figures 7A, 7B et 7C annexées représentent d'autres exemples plus spécifiques de monomères triazine conformes à l'invention, sous forme sulfonés, de formules respectives 111-1-B, III-2-B et III-3-B, dans lesquelles X₁ et X₂ sont identiques, Z₁ et Z₂ correspondent au Fluor, et trois des groupes phényle (ici, à titre d'exemple, les groupes phényle Ar₁, Ar₂ et Ar₃) sont substitués par un groupe sulfonate SO₃M (M représentant un cation de métal alcalin, préférentiellement Na⁺ ou K⁺) ; il s'agit donc ici plus précisément, et respectivement, des sels de métal alcalin de 2,4-bis-{(4-fluoro-phénylsulfanyl phényl)-(3-sulfonatophényl)-6-(m-sulfonatophényl)}-[1,3,5]-triazine, de 2,4-bis-{(4-fluoro-phénylsulfoxy phényl)-(3-sulfonatophényl)-6-(m-sulfonatophényl)}-[1,3,5]-triazine et de 2,4-bis-{(4-fluoro-phénylsulfonyl phényl)-(3-sulfonatophényl)-6-(m-sulfonatophényl)}-[1,3,5]-triazine.

Selon un autre exemple de réalisation particulièrement préférentiel, les groupes Z₁ et Z₂ correspondent au chlore, c'est-à-dire que le monomère triazine de l'invention répond alors à la formule :

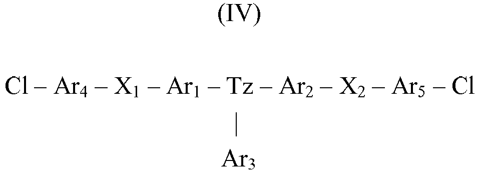

Ainsi, dans le cas plus préférentiel où X₁ et X₂ sont identiques, le monomère triazine de l'invention de formule (I) répond à l'une des trois formules III-1, III-2 et III-3 représentées précédemment respectivement aux figures 5A, 5B et 5C annexées, mais dans lesquelles le chlore remplace le fluor. En particulier, le monomère triazine de l'invention répond à l'une des formules III-1-A, III-2-A, III-3-A, III-1-B, III-2-B et III-3-B, représentées précédemment respectivement aux figures 6A, 6B, 6C, 7A, 7B et 7C annexées, mais dans lesquelles le chlore remplace le fluor.

Le monomère triazine conforme à l'invention précédemment décrit est avantageusement utilisable pour la synthèse de polymères triazine pouvant constituer, sous forme sulfonés, un électrolyte (ou membrane, ce qui est équivalent) dans une pile à combustible. Par polymère doit être entendu tout homopolymère ou copolymère, notamment copolymère à blocs, comportant des unités structurelles issues du monomère de l'invention.

On entend par "monomère sulfoné" ou "polymère sulfoné", par définition et de manière bien connue, un monomère ou polymère porteur d'un ou plusieurs groupes sulfoniques (-SO₃H), sulfonates (-SO₃M), ou mélanges de tels groupes, M représentant un cation de métal alcalin choisi préférentiellement parmi lithium (Li), césium (Cs) sodium (Na) et potassium (K), plus préférentiellement parmi sodium (Na) et potassium (K). On rappellera brièvement ici que ce sont les groupes sulfoniques qui dans une pile PEM assurent la conductivité protonique du polymère utilisé comme membrane. De préférence, le groupe sulfonique ou sulfonate est porté par au moins un groupe phényle ou phénylene ou, le cas échéant, par au moins un substituant des groupes phényle ou phénylene.

Les figures 8 à 14 annexées représentent plusieurs exemples de polymères synthétisables à partir de monomères triazine de formule (I) conformes à l'invention, ainsi que divers schémas de synthèse possibles de ces polymères à partir de ces monomères.

Le polymère triazine (ci-après dénommé "Polymère 1") tel que représenté à la figure 8 (sous forme sulfoné) est constitué de deux types d'unités structurelles à base triazine reliées entre elles par des ponts éther (-O-). Ce Polymère 1 peut être préparé par polycondensation d'un monomère conforme à l'invention noté A1 à la figure 8 avec un second monomère noté B1 (disulfoné, non conforme à l'invention), en présence d'une base et d'un solvant organique, selon un mode opératoire qui sera décrit en détail plus loin. On note que le monomère A1 correspond au monomère triazine de formule (II-3) précédemment décrit (Fig. 2C).

Le polymère triazine (ci-après dénommé "Polymère 2") tel que représenté à la figure 9 (également sous forme sulfoné) est lui aussi constitué de deux types d'unités structurelles à base triazine reliées entre elles par des ponts éther (-O-). Ce Polymère 2 peut être préparé par polycondensation d'un monomère (sulfoné) conforme à l'invention noté A2 à la figure 9 avec un second monomère (non conforme à l'invention) noté B2, en présence d'une base et d'un solvant organique. On note que le monomère A2 correspond au monomère triazine de formule (II-3-A) précédemment décrit (Fig. 3C).

Le polymère triazine (ci-après dénommé "Polymère 3") tel que représenté à la figure 10 (également sous forme sulfoné) est lui aussi constitué de deux types d'unités structurelles à base triazine reliées entre elles par des ponts éther (-O-). Ce Polymère 3 peut être préparé par polycondensation d'un monomère (sulfoné) conforme à l'invention noté A3 à la figure 10 avec un second monomère (disulfoné, non conforme à l'invention) noté B3, en présence d'une base et d'un solvant organique. On note que le monomère noté A3 (pour rendre homogènes les références dans les différentes figures) correspond également au monomère noté A2 de la figure précédente.

Les polymères triazine (ci-après dénommés 'Polymère 4A" et "Polymère 4B") de la figure 11 (également sous forme sulfonés) sont eux aussi constitués de deux types d'unités structurelles à base triazine reliées entre elles par des ponts éther (-O-). Ces Polymères 4A et 4B peuvent être préparés par copolymérisation d'un monomère conforme à l'invention noté A4 avec deux autres monomères non conformes à l'invention notés B4 et C4 (monomère C4 disulfoné) sur la figure 11, en présence d'une base et d'un solvant organique appropriés, comme précédemment pour les Polymères 1, 2 ou 3. Le premier polymère (Polymère 4A) ainsi obtenu est ensuite oxydé par le peroxyde d'hydrogène (eau oxygénée) pour obtention du polymère final (Polymère 4B). Les monomères B4 et C4 sont connus, disponibles commercialement. Le monomère A-4 conforme à l'invention est préparé selon un mode opératoire qui sera décrit en détail ultérieurement ; ce monomère A4 correspond au monomère triazine de formule (II-1) précédemment décrit (Fig. 2A).

Le polymère triazine (ci-après dénommé "Polymère 5") de la figure 12 (également sous forme sulfoné) est constitué quant à lui des mêmes unités structurelles à base triazine reliées entre elles par des ponts éther (-O-). Ce Polymère 5 peut être préparé par polycondensation d'un monomère (sulfoné) conforme à l'invention noté A5 à la figure 12 avec un second monomère également conforme à l'invention noté A5bis. Le monomère noté A5 (pour rendre homogène les références dans les figures) est en fait identique aux monomères triazine notés A2 et A3 aux figures précédentes ; le monomère A5bis correspond au monomère triazine de formule (III-3) précédemment décrit (Fig. 5C).

Le polymère triazine (ci-après dénommé "Polymère 7") de la figure 13 (également sous forme sulfoné) est lui aussi constitué de deux types d'unités structurelles à base triazine reliées entre elles par des ponts éther (-O-). Ce Polymère 7 peut être préparé par polycondensation d'un monomère conforme à l'invention noté A7 à la figure 13 avec un second monomère (disulfoné) non conforme à l'invention noté B7, en présence d'une base et d'un solvant organique, selon un mode opératoire qui sera décrit en détail plus loin. Le monomère noté A7 (pour rendre homogène les références dans les figures) est identique au monomère triazine noté A4 à la figure 11 précédente, le monomère B7 étant quant à lui identique aux monomères B1 et B3 des figures 8 et 10.

Le polymère triazine (ci-après dénommé "Polymère 8") de la figure 14 (également sous forme sulfoné) est lui aussi constitué de deux types d'unités structurelles à base triazine reliées entre elles par des ponts éther (-O-). Ce Polymère 8 peut être préparé par polycondensation d'un monomère conforme à l'invention noté A8 à la figure 14 avec un second monomère (disulfoné) non conforme à l'invention noté B8, en présence d'une base et d'un solvant organique, selon un mode opératoire qui sera décrit en détail plus loin. Le monomère noté A8 (pour rendre homogène les références dans les figures) est identique aux monomères triazine notés A4 et A7 aux figures 11 et 13 précédentes. Ici, le polymère triazine comporte des extrémités de chaînes bloquées par des groupements bloquants benzophénone (notés B à la figure 14), hydrophobes et stériquement encombrants, destinés à réduire la solubilité du polymère dans l'eau.

### V. EXEMPLES DE REALISATION DE L'INVENTION

Les essais qui suivent décrivent tout d'abord en détail la synthèse des monomères A1, B1 (aussi dénommé B7), A7 (aussi dénommé A4 et A8) et B8, puis celle des Polymère 1, Polymère 7 et Polymère 8.

Puis, le Polymère 1 est caractérisé et testé comme une membrane conductrice de protons dans une pile à combustible du type PEM.

Dans la présente description, sauf indication expresse différente, tous les pourcentages (%) indiqués sont des % en masse.

### V-1. Synthèse du monomère A1

Le monomère A1 est la 2,4-[(4-hydroxy-phénylsulfonyl phényl)]-6-(phényl)-[1,3,5]-triazine, dont la formule II-3 (déjà reproduite aux figure 2C et figure 8) est la suivante :

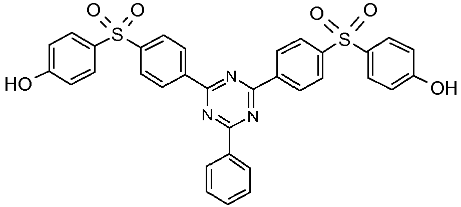

Ce monomère A1 (ou Composé 3 à la Fig. 15) a été préparé selon le mode opératoire schématisé à la figure 15, en trois étapes successives, comme détaillé ci-après.

### V-1-A) Etape 1

Lors d'une première étape, on prépare le Composé 1 ou 2,4-bis-(p-fluorophényl)-6-phényl-[1,3,5]-triazine, selon le mode opératoire qui suit et schématisé à la figure 15A.

Ce mode opératoire bien que différent s'inspire du procédé de synthèse de triphényl-triazines chlorées tel que décrit dans la publication de Spencer R.D. & Beggs B.H, "Determination of Four Closely Related Triaryl-s-Triazines by Infrared Spectroscopy", Anal. Chem. 1963, 31(11), 1633-1636.

Un ballon rond tricol de 500 ml équipé d'un barreau magnétique, d'un réfrigérant, d'un thermomètre, est séché à l'aide d'un pistolet à air chaud (l'appareillage est mis sous vide). 67,8 g de p-fluorobenzonitrile (0,56 mol) (Fluorochem 99%), 36,0 g de chlorure d'ammonium (0,68 mol), 34,0 g de chlorure d'aluminium (0,26 mol) et 32,0 g de chlorure de benzoyle (0,22 mol) sont placés dans le ballon sous azote. Le ballon est plongé dans un bain d'huile chauffé à 158°C et laissé toute la nuit à 150°C (température à l'intérieur du ballon de réaction), un léger flux d'azote au dessus du mélange réactionnel.

Le produit de réaction est refroidi à température ambiante (environ 23°C) et hydrolysé en additionnant 300 g de glace et 60 g de HCl 36%. Le solide est filtré, puis dispersé dans l'eau et lavé jusqu'à l'obtention d'un pH neutre. Le solide blanc est agité dans 500 ml de méthanol chauffé à reflux pendant 30 min, puis on laisse refroidir à température ambiante. Pour finir le produit est filtré et séché à 60°C sous vide.

On obtient ainsi 26,6 g (rendement 35%) de Composé 1, présentant un point de fusion (selon DSC) de 254,5°C.

L'analyse RMN donne les résultats suivants :
¹H NMR, 500 MHz (*CD₂Cl₂*): 7.30 - 7.34 (m, 4H), 7.62 - 7.65 (m, 2H), 7.68 - 7.70 (m, 1H), 8.79 - 8.80 (d, 2H), 8.82 - 8.85 (m, 4H).

### V-1-B) Etape 2

Lors d'une seconde étape, on prépare le Composé 2 conforme à l'invention (aussi dénommé monomère A4 - voir par exemple Fig. 11) ou 2,4-[(4-hydroxy-phénylsulfanyl phényl)]-6-(phényl)-[1,3,5]-triazine, selon le mode opératoire qui suit et schématisé à la figure 15B.

Le 4-hydroxythiophénol (ou 4-HTP) (99%, Acros) est conservé sous azote et sous forme solide. Le Composé 1 et K₂CO₃ sont séchés séparément toute la nuit à 150°C sous vide. Un barreau magnétique est placé dans un ballon rond de 21 (équipé d'un réfrigérant, d'un thermomètre et d'une entrée/sortie d'azote). L'appareillage est placé sous vide et séché. Une valve à deux voies est utilisée pour remplacer le vide par l'azote et purger continuellement avec le gaz inerte pendant l'addition des réactifs.

Le Composé 1 (9,13 g soit 26,44 mmol) et K₂CO₃ anhydre en poudre (9,69 g soit 1,2 eq. par rapport au 4-HTP) sont additionnés, pendant qu'il sont encore chauds (en sortie de séchage), dans l'appareillage purgé à l'azote. Ceci est suivi par l'addition de 750 ml de DMSO anhydre. La suspension obtenue est ensuite purgée pendant au moins 15 min avec un courant d'azote à l'intérieur de la solution.

La quantité requise de 4-HTP (7,45 g ou 58,42 mmol, soit 2,2 eq.), sous forme liquide, est transférée à l'aide d'une seringue en plastique de 10 ml, pesée directement à l'intérieur de la seringue et injectée dans le mélange réactionnel. Une fois que tous les réactifs sont additionnés, l'azote est purgé en continu au-dessus de la solution. Le mélange est chauffé à 100°C avec une agitation constante toute la nuit (20 heures), puis on laisse refroidir à température ambiante.

La purification du produit ne peut être faite en une seule étape : environ 250 ml de fraction aliquote de la réaction sont prélevés et versés dans une ampoule à extraction (3 litres) contenant 2,6 litres d'acétate d'éthyle/eau (rapport pondéral 1/1). Le reste du produit est gardé sous un flux constant d'azote. Le mélange placé dans l'ampoule à extraction est secoué (la couleur change du orange au jaune-citron) et le produit désiré est extrait dans la phase acétate d'éthyle (la phase DMSO/H₂O contient seulement des traces du produit recherché). La phase organique est lavée avec 100 ml d'une solution de NaHCO₃, étape suivie par un lavage avec 100 ml de H₂O ; la phase organique est ensuite séchée avec du MgSO₄ anhydre. Le procédé est répété deux fois avec les deux autres aliquotes restants de 250 ml du mélange réactionnel.

La phase d'acétate d'éthyle est évaporée à l'aide de l'évaporateur rotatif, il reste un liquide visqueux légèrement orange comme du miel (contenant une petite quantité de DMSO). Les résidus de DMSO sont retirés à 100°C sous pression réduite. Une petite quantité d'acétone (10 ml) est ajoutée suivie de 40 ml de diéthyéther. Le solide devient immédiatement blanc crème, il est filtré sur un filtre en céramique. Le thiol résiduel est enlevé du produit réactionnel par chromatographie sur colonne en utilisant hexane/CH₂Cl₂/acétate d'éthyle/méthanol (rapports pondéraux 4/2/1/1) comme phase mobile.

On obtient ainsi 13,1 g (soit un rendement d'environ 89%) du Composé 2.

L'analyse RMN donne les résultats suivants :
*¹H NMR (500 MHz) DMSO-d₆: 6.93-6.95 (d, 4H), 7.17-719 (d, 4H), 7.42-7.44 (d, 4H), 7.58-7.60 (m, 2H), 7.65-7.68 (m, 1H), 8.49-8.50 (d, 4H), 8.61-8.63 (d, 2H), 10.04 (s, 2H).*

La masse moléculaire du produit telle que mesurée par spectrométrie de masse "MALDI" (*Matrix-assisted Laser Desorption*/*Ionisation*) (mode positif ; matrice dithranol) est égale à 558,1 (valeur théorique calculée égale à 557,7).

### V-1-C) Etape 3

Enfin, lors d'une troisième et dernière étape, on prépare le Composé 3 conforme à l'invention (monomère A1) selon le mode opératoire qui suit et schématisé à la figure 15C.

Un ballon rond tricol de 250 ml est équipé d'un barreau magnétique, d'un thermomètre, d'un réfrigérant et d'une ouverture servant à l'ajout des réactifs. Une suspension est préparée par ajout de 6,69 g de Composé 2 (12 mmol) dans 150 ml d'acide acétique glacial. Une fois le réactif ajouté, la suspension est chauffée à 70°C, le réactif se dissout donnant une légère coloration jaune transparente. Par la suite, 18,0 g de peroxyde d'hydrogène à 50% (264 mmol) sont introduits goutte à goutte dans la réaction (aucune exothermie n'est observée). La solution est chauffée à reflux (100°C) pendant 1 heure (coloration légèrement jaune). Une chromatographie sur couche mince (plaque en silice) dans CH₂Cl₂/acétate d'éthyle/méthanol (rapport pondéraux 3/1/1) permet de suivre la consommation du réactif pendant la réaction (la fluorescence bleue de la triazine à 325 nm disparaît avec l'oxydation).

Par la suite, 50 ml d'acide acétique sont éliminés par distillation à pression réduite (vide généré par trompe à eau). Après distillation, lors du refroidissement, des cristaux blancs commencent à précipiter dans la solution dès que la température devient inférieure à 80°C. La solution est laissée toute la nuit à température ambiante afin que le produit cristallise dans l'acide acétique. Puis l'acide acétique est enlevé par filtration et le produit final blanc est lavé avec 300 ml d'eau distillée. Ensuite, environ 18 g de produit humide ainsi obtenu sont transférés dans un ballon à fond rond et 75 ml d'eau distillée sont ajoutés, le tout agité pendant environ 15 min. Le produit est ensuite filtré et lavé jusqu'à une valeur de pH neutre. Le produit encore humide est séché pendant 2 h à 60°C sous vide, puis à 100°C toute la nuit (environ 12 h) sous vide.

La purification est faite par chromatographie sur colonne en utilisant CH₂Cl₂/acétate d'éthyle/méthanol (3/1/1) comme phase mobile.

Le pic endothermique se situe à environ 294°C (1^{er} passage ("*run*") de DSC). On note que le monomère polymérise immédiatement lors du second "*run*" de DSC ; la température de transition vitreuse (Tg) du polymère ainsi formé se situe à environ 145°C.

On obtient ainsi 5,35 g (rendement d'environ 80%) du Composé 3 ou monomère A1.

La figure 16 reproduit le spectre RMN ¹H (360 MHz) du monomère A1 conforme à l'invention ainsi obtenu, dissous dans DMSO-*d₆*.

L'analyse RMN donne les résultats suivants :
*¹H NMR (360 MHz) DMSO-d₆ : 6.99-7.01 (d, 4H),* 7.62-7.67 *(m, 2H), 7.72 (t, 1H), 7.87-7.90 (d, 4H), 8.13-8.16 (d, 4H), 8.66-8.68 (d, 2H), 8.80-8.83 (d, 4H), 10.77 (s, 2H).*

Enfin, la masse moléculaire du produit telle que mesurée par spectrométrie de masse "ESI" *(Electrospray Ionisation)* (mode négatif ; mélange eau/acétone 1/1) est égale à 620,7 (valeur théorique calculée égale à 621,7).

### V-2. Synthèse du monomère B1

Le monomère B1, pour rappel non conforme à la présente invention, est le 3,3'-bis (4-fluorophénylsulfonyl) perfluorobutane disulfoné, dont la formule (déjà reproduite à la figure 8) est la suivante :

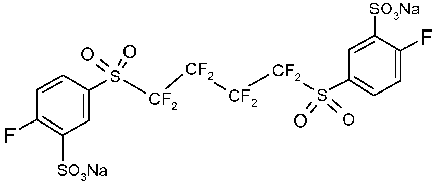

Ce monomère B1 (ou Composé 6 à la Fig. 17) a été préparé selon le mode opératoire schématisé à la figure 17, en trois étapes successives, comme détaillé ci-après.

### V-2-A) Etape 1

Lors d'une première étape, on prépare le Composé 4 ou 1,4-bis-(4-fluorophénylthio)-perfluorobutane, selon le mode opératoire qui suit et schématisé à la figure 17A.

Ce mode opératoire, bien que différent, s'inspire du procédé de synthèse de polyéthersulfones fluorés tel que décrit dans la publication de Feiring A.E., Wonchoba E.R. & Arthur R.D., "Fluorinated Poly(ether Sulfone)s", J. Polym. Sci., Part A: Pol. Chem, 1990, 38, 2809-2818.

Un mélange de méthanolate de sodium (13,64 g) (Fluka, 97%) et de 4-fluorothiophénol (31,70 g) (Fluorochem, 99%) dans 200 ml de méthanol anhydre est chauffé à reflux pendant 60 min. Après distillation du méthanol, le solide blanc est gardé sous azote dans l'appareillage à température ambiante.

A une solution de 37,0 g de sel de 4-fluorophénylthiolate de sodium (244,83 mmol) dans 170 ml de DMF anhydre, sous azote et refroidi à 0°C, sont additionnés 51,0 g de 1,4-diiodoperfluorobutane (0,110 mol) (Apollo Scientific, 98%) ; une exothermie se produit et la température atteint 40°C. La solution obtenue est maintenue à 40°C et agitée (environ 12 heures), elle est ensuite chauffée à 60°C pendant une heure. La solution une fois revenue à température ambiante est diluée avec 60 ml d'eau, puis concentrée à l'aide de la pompe à vide afin de retirer 100 ml de solvant. La solution restante est diluée avec de l'eau, la phase inférieure est séparée et lavée avec de l'eau. Le produit est distillé sous vide à 120°C. Après avoir éliminé les impuretés, un liquide incolore est récupéré, soit 37,9 g (75,6%). Les traces d'impuretés restantes (thiol) sont éliminées par chromatographie sur colonne en utilisant de l'hexane comme phase mobile, donnant un produit ressemblant à de la cire solide et transparente à température ambiante. Le point de fusion du produit est égal à environ 50°C (mesuré par DSC).

On obtient ainsi le Composé 4, de formule :

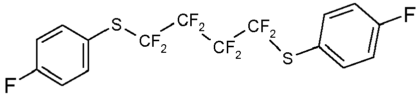

L'analyse RMN donne les résultats suivants :
*¹H NMR, 500 MHz (CDCl₃): 7.09 -* 7.12 *(m, 4H), 7.62 - 7.65 (m, 4H).*

### V-2-B) Etape 2

Puis, au cours d'une seconde étape, on prépare le Composé 5 ou 1,4-bis-(4-fluorophénylsulfonyl)-perfluorobutane, selon le mode opératoire qui suit et schématisé à la figure 17B.

Un ballon rond bicol d'un litre, équipé d'un réfrigérant, d'un barreau magnétique, et d'une entrée d'azote, est chargé avec 31,80 g (80,0 mmol) de Composé 4, 350 ml d'acide acétique glacial et 65,4 g (soit 413 mmol) de KMnO₄ (5,9 eq.). Après 10 min d'agitation à température ambiante, la solution est refroidie entre 0°C et 5°C puis 35 ml d'acide sulfurique concentré sont ajoutés goutte à goutte pendant le refroidissement avec le bain de glace (température comprise entre 0 et 5°C, pendant 5 heures). Le mélange réactionnel est agité toute la nuit à température ambiante, puis versé dans 3,5 litres d'eau distillée. Le produit est extrait avec 7 litres de chloroforme. Le MnO₂ hydrolysé est filtré à chaque fois sur un papier filtre plus un filtre textile. Le solvant (chloroforme/acide acétique) est retiré à l'aide de l'évaporateur rotatif à 50° C. Puis le produit est dissous dans 1 litre de chloroforme. La phase organique est ensuite lavée successivement avec 200 ml d'une solution saturée de NaHCO₃, puis avec 200 ml d'eau distillée, enfin séchée avec MgSO₄. Le solvant est éliminé à l'évaporateur rotatif, puis le produit est purifié par chromatographie sur colonne en utilisant un mélange hexane/acétate d'éthyle/méthanol (15/3/2) comme éluant afin d'obtenir le Composé 5.

Le produit sous forme de cristaux blancs est séché toute la nuit à 60°C sous vide. Il est ensuite recristallisé dans l'acétone afin d'obtenir des cristaux transparents. L'analyse DSC révèle un point de fusion d'environ 127°C.

On obtient ainsi 32,6 g (rendement 90%) de Composé 5, de formule :

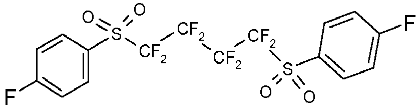

L'analyse RMN donne les résultats suivants :
*¹H NMR, 500 MHz (CDCl₃): 7.36 - 7.39 (m, 4H), 8. 07 - 8.10 (m, 4H).*

### V-2-C) Etape 3

Enfin au cours d'une troisième et dernière étape, on prépare le Composé 6 ou monomère B1 (3,3'-bis(4-fluorophénylsulfonyl)-perfluorobutane disulfoné), selon le mode opératoire qui suit et schématisé à la figure 17C.

Le Composé 5 (5,0 g soit 9.65 mmol) est placé dans un ballon rond de quatre cols séché au pistolet à air chaud puis mis sous azote (barreau magnétique recouvert de verre). L'acide sulfurique concentré (23,6 g) est ensuite ajouté en utilisant un cylindre gradué en verre préalablement séché. La plupart du composé ne se solubilise pas dans l'acide sulfurique à température ambiante (la solution devient légèrement violette). Finalement, 20,06 g d'oléum (produit Merck à 65% SO₃) sont ajoutés en utilisant une ampoule à addition graduée séchée au préalable. Le bulleur de sortie de gaz est rempli d'acide sulfurique concentré et les produits gazeux sont purgés à travers une trappe vide puis à travers une trappe remplie de NaOH à 10%. Le milieu réactionnel est chauffé à 120°C (température du bain d'huile de 128°C) avec un flux moyen d'azote circulant au-dessus de la solution. La réaction est poursuivie à 120°C durant toute la nuit (environ 12 h).

Une fois la sulfonation terminée, le mélange réactionnel est refroidi à 90 °C, puis versé encore chaud dans 250 g de glace. On laisse le tout sous agitation ; une fois que toute la glace a fondu, 15 g de NaCl sont ajoutés précipitant le monomère disulfoné. Le précipité est ensuite filtré puis séché à 80°C sous vide. Le produit sec est ensuite mélangé avec 250 ml d'eau distillée et chauffé jusqu'à 90°C. Une fois que tout le produit s'est dissous, le pH est ajusté à 7,0 en ajoutant NaOH (aqueux) à 1%. La solution est refroidie à température ambiante ; à ce moment-là, la plupart du produit a précipité. On sépare le produit blanc de la phase aqueuse par filtration. Le produit restant dans la phase aqueuse est précipité en ajoutant 15 g de NaCl. Le produit est filtré et séché toute la nuit à 150°C sous vide. Aucune autre purification n'est nécessaire.

On obtient ainsi 5,92 g (rendement 85%) du monomère B1, de formule :

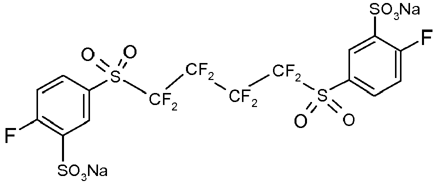

L'analyse RMN donne les résultats suivants :
¹H NMR, 500 MHz (*DMSO-d₆*): 7.67 - 7.70 (m, 2H), 8.20 - 8.23 (m, 2H), 8.29 - 8.31 (m, 2H).

La figure 18 reproduit le spectre RMN ¹H (500 MHz) du monomère B1 ainsi obtenu, dissous dans DMSO-*d₆*.

Le produit apparaît pur selon une analyse par chromatographie en couche mince "TLC" (*Thin Layer Chromatography*) sur plaques silice, utilisant un mélange dichlorométhane/éthyl acétate/méthanol (7:7:6).

Enfin, la masse moléculaire du produit telle que mesurée par spectrométrie de masse "ESI" (*Electrospray Ionisation*) (mode négatif (M⁻ - Na⁺) ; mélange eau/acétone 1/1) est égale à 698,8 (valeur théorique calculée égale à 699,5).

### V-3. Synthèse du monomère B8

Le monomère B8, pour rappel non conforme à la présente invention, est le 1,4-bis-(4-fluorobenzophénone)-perfluorobutane disulfoné, dont la formule est la suivante :

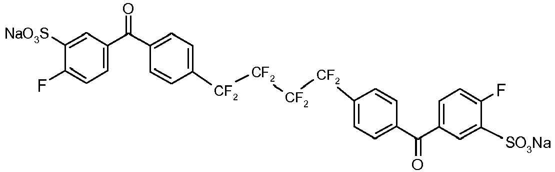

Ce monomère B8 (ou Composé 9) a été préparé selon le mode opératoire schématisé à la figure 19, en trois étapes successives, comme détaillé ci-après.

### V-3-A) Etape 1

Lors d'une première étape, on prépare le Composé 7 ou 4-iodo-4'-fluorobenzophénone, conformément à la figure 19A.

Dans un ballon préalablement séché de 250 ml sont ajoutés le 4-iodobenzoyl chlorure (30 g soit 112,6 mmol), le chlorure d'aluminium (15,0 g soit 112,7 mmol) et le fluorobenzène (21,7g soit 225, 8 mmol). Le mélange est agité à température ambiante sous un léger flux d'azote toute la nuit. Le lendemain, un solide est apparu et l'agitation n'est plus possible. On additionne alors 20 ml de fluorobenzène supplémentaires et les réactifs sont mélangés à 40°C (température à l'intérieur du ballon) pendant 3 h. L'appareillage est placé à 40°C sous vide (trompe à eau) et l'excès de fluorobenzène est distillé (pendant 30 min).

Directement dans le ballon de réaction sont ajoutés 200 g de glace, puis immédiatement 60 ml de HCl à 37%. Le produit solide ainsi obtenu est réduit en poudre dans un mortier en céramique, puis agité dans l'eau jusqu'à l'obtention d'une poudre blanche finalement séparée de la solution HCl par filtration (papier filtre) et lavée jusqu'à pH neutre. Le solide est séché à température ambiante (23°C) à l'aide de la trompe à eau, puis mélangé à 200 ml d'éthanol et finalement chauffé à 60°C (température à l'intérieur du ballon) jusqu'à ce que le tout soit dissous. Le composé est finalement précipité en refroidissant l'éthanol à température ambiante.

Le produit final (environ 30 g) est purifié par chromatographie sur silice (300 g) en utilisant un mélange hexane/acétate d'éthyle (rapport pondéral 16/4) comme phase mobile. Le produit est séparé de la phase mobile à l'évaporateur rotatif et séché à 80°C toute la nuit (sous vide). Le produit final de couleur crème (25 g) se révèle pur par analyse RMN et chromatographie TLC dans le mélange hexane/acétate d'éthyle (rapport 16/4), avec un point de fusion (mesuré par DSC) d'environ 137°C.

On obtient ainsi le Composé 7, de formule :

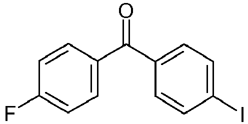

L'analyse RMN donne les résultats suivants :
*¹H NMR, 500 MHz (CD₂Cl₂): 7.17 - 7.20 (m, 2H), 7.48-7.50 (m, 2H), 7.80-7.82 (m, 2H),* 7.87-7.89 *(m, 2H).*

### V-3-B) Etape 2

Puis, au cours d'une seconde étape, on prépare le Composé 8 ou 1,4-bis-(4-fluorobenzo-phénone)-perfluorobutane, selon le mode opératoire qui suit et schématisé à la figure 19B.

Dans un ballon de 500 ml à 4 cols préalablement séché sont introduits 17,0 g de 4-iodo-4'-fluorobenzophénone (soit 52,13 mmol), 2,0 g de 2,2'-bipyridyl (soit 12,83 mmol) suivis de 11,83 g de 1,4-diiodoperfluorobutane (soit 26,06 mmol) et 150 ml de DMSO anhydre. Par la suite, 6,60 g de poudre de cuivre sont ajoutés et la solution est chauffée à 65°C (le bain d'huile est réglé à 74°C) pendant 5 h sous agitation constante et courant d'azote.

Le mélange réactionnel est refroidi à température ambiante puis versé dans 500 ml d'eau froide ; le produit précipite, puis il est filtré et dissous avec 1 litre de dichlorométhane. La phase organique est ensuite séchée avec Na₂SO₄ anhydre. Le produit final est purifié par chromatographie sur silice (300 g) dans un mélange dichlorométhane/cyclohexane (1/1).

On obtient ainsi le Composé 8 sous forme d'une poudre blanche, de formule :

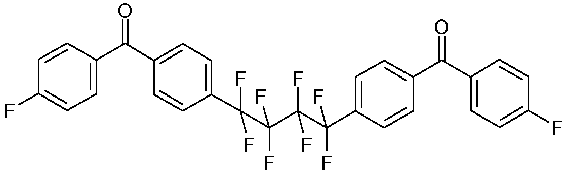

L'analyse RMN donne les résultats suivants :
*¹H NMR, 500 MHz (CD₂Cl₂):* 7.19 - 7.23 *(m, 2H),* 7.73-7. 75 *(d, 4H),* 7.83-7.87 *(m, 8H). ¹⁹F NMR,* 471.3 *MHz (CDCl₃): 105.04 (m, 2F), 111.44-111.50 (d, 4F), 121.49-121.55 (m, 4F).*

La température de fusion du produit (mesurée par DSC) est égale à environ 222°C.

### V-3-C) Etape 3

Enfin au cours d'une troisième et dernière étape, on prépare le Composé 9 ou 1,4-bis-(4-fluorobenzophénone)-perfluorobutane disulfoné, selon le mode opératoire qui suit et schématisé à la figure 19C.

Le Composé 8 (2,5g soit 4,18 mmol) est placé dans un ballon de 50 ml à 4 cols préalablement séché l'aide d'un pistolet à air chaud et mis sous flux d'azote. 6 g d'acide sulfurique (distillé deux fois, Sigma-Aldrich) et 10 g d'oléum (65%, Merck) sont additionnés directement au solide. Le milieu réactionnel devient immédiatement foncé. La sortie des produits gazeux est purgée dans une trappe en verre vide suivie d'une trappe remplie de NaOH 30%. Le milieu réactionnel est chauffé à environ 130°C (environ 138°C dans le bain d'huile) pendant 4 h sous un flux moyen d'azote circulant au-dessus de la solution.

Une fois la sulfonation terminée, on laisse le mélange réactionnel se refroidir à température ambiante puis on le verse dans 63 g de glace et on laisse sous agitation. Une fois toute la glace fondue, 6,25g de NaCl sont additionnés. La solution est chauffée à 100°C puis refroidie à température ambiante afin que le monomère sulfoné précipite. Le précipité est ensuite dissous de nouveau dans 15 ml d'eau et chauffé à nouveau à 100°C pour le repasser sous forme liquide. Une fois que tout le produit s'est dissous, le pH est ajusté à 7,0 en additionnant goutte à goutte du NaOH (aq.) 10%. On laisse la solution se refroidir à température ambiante. Le solide blanc-crème ainsi obtenu est séparé de la phase aqueuse par filtration. Le produit est séché à 150°C toute la nuit (sous vide).

On obtient ainsi le Composé 9 (monomère B8), de formule :

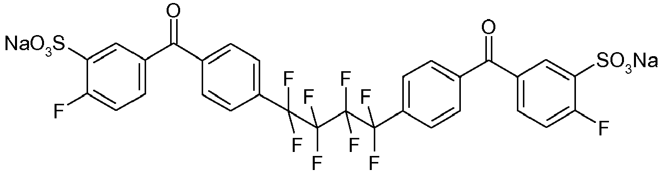

La figure 20 reproduit le spectre RMN ¹H (500 MHz) du monomère B8 ainsi obtenu, dissous dans DMSO-*d₆*.

L'analyse RMN donne les résultats suivants :
*¹H NMR, 500 MHz (DMSO-d₆):* 7.35 - 7.39 *(m, 2H), 7.80-7.83 (m, 2H),* 7.87-7.937 *(m, 8H), 8.09-8.10 (d, 1H), 8.11-8.12 (d, 1 H).*

Enfin, la masse moléculaire du produit telle que mesurée par spectrométrie de masse "ESI" (*Electrospray Ionisation*) (mode négatif ; mélange eau/acétone 1/1) est égale à 778,9 (valeur théorique calculée égale à 779,6).

### V-4. Synthèse du Polymère 1

Cet exemple décrit de manière détaillée la synthèse, à partir des monomères A1 (conforme à l'invention) et B1 (non conforme à l'invention) précédemment décrits, du Polymère 1 sous forme sulfoné, bloqué par des groupements benzophénone, tel qu'il est représenté à la figure 21.

Le monomère A1 est séché à 60°C sous vide toute la nuit. Le monomère B1 et Na₂CO₃ sont séchés séparément à 150 °C sous vide toute la nuit. Puis les trois composés sont mélangés et séchés à 160°C sous vide pendant une heure. La copolymérisation des monomères A1 et B1 se fait dans un ballon rond tricol de 100 ml. Le ballon est équipé d'une entrée d'azote, d'un thermomètre, d'un agitateur magnétique et d'un séparateur "Dean Stark" surmonté d'un réfrigérant. Les parties en verre de l'appareillage sont séchées sous vide en utilisant un pistolet à air chaud pour atteindre une température d'au moins 100°C dans la ballon de réaction.

Le ballon de réaction est chargé avec le monomère A1 (1,89 g soit 3,04 mmol ou 1 eq.), le monomère B1 (2,20 g soit 3,04 mmol ou 1 eq.), le carbonate de sodium anhydre (0,97 g soit 9,13 mmol ou 3 eq.), du N,N-diméthylacétamide anhydre (20 ml) et du toluène (4,0 ml, agent azéotropique). Le ballon de réaction est chauffé à 100°C dans un bain d'huile pendant une heure (distillation azéotropique). La vanne de circulation du toluène est ensuite fermée et le toluène est distillé à 100°C. La température du bain d'huile est ensuite augmentée à environ 148°C et les résidus de toluène sont éliminés par distillation pendant 60 min supplémentaires de façon à ce que tout le toluène soit retiré de la réaction et que la température augmente à 140°C à l'intérieur du ballon. Le toluène est vidangé du séparateur "Dean Stark", la température du bain d'huile est augmentée à environ 159°C et maintenue à cette valeur toute la nuit. Après 20 h environ, la température du bain d'huile est augmentée à environ 168°C (environ 152°C à l'intérieur du ballon) et la polymérisation continue pendant 4 heures. La température de la réaction est descendue à environ 130°C à l'intérieur du ballon, en retirant le ballon du bain d'huile. 8 mg de 4-fluorobenzophénone dissous dans 5 ml de N,N-diméthylacétamide anhydre, sont ensuite ajoutés à la réaction à l'aide d'une seringue. Le ballon est replacé dans le bain d'huile et la réaction continue à environ 152°C (168°C dans le bain d'huile) pour une période de 4 h supplémentaires. On laisse refroidir le mélange réactionnel à température ambiante, le polymère est ensuite versé dans 500 ml d'isopropanol. Le précipité fibreux est récupéré par filtration et lavé avec de l'isopropanol et de l'eau jusqu'à l'obtention d'un pH neutre (lavage des sels résiduels). Le produit est ensuite séché à 60°C sous vide toute la nuit. La purification est faite par précipitation du polymère dissous dans du N,N-diméthylacétamide, versé goutte à goutte dans du diéthyléther sous une agitation constante.

La formule du Polymère 1 ainsi obtenu, sous forme sulfoné et bloqué benzophénone, est représentée à la figure 21, ainsi que son spectre RMN ¹H (500 MHz), dissous dans DMSO-*d₆*.

### V-5. Synthèse du Polymère 7

Cet exemple décrit de manière détaillée la synthèse du Polymère 7 selon on procédé déjà commenté à la figure 13, à partir du Composé 2 (conforme à l'invention) et du Composé 6 (non conforme à l'invention) précédemment décrits, ce Polymère 7 étant obtenu ici d'une part sous forme sulfoné et d'autre part bloqué par des groupements benzophénone, tel qu'il est représenté à la figure 22.

Le monomère noté A7 ou A4 (Composé 2) est séché à 80 °C sous vide toute la nuit. Le monomère noté B7 ou B1 (Composé 6) et Na₂CO₃; sont séchés séparément à 150°C, mélangés puis le tout est séché à 160°C sous vide pendant une heure. La copolymérisation des monomères A7 et B7 se fait dans un ballon rond tricol de 100 mL. Le ballon est équipé d'une entrée d'azote, d'un thermomètre, d'un agitateur magnétique et d'un séparateur "Dean Stark" surmonté d'un réfrigérant. Les parties en verre de l'appareillage sont séchées sous vide.

Pour une disulfonation à 50% mol, le ballon est chargé avec le monomère A7 (1,695 g soit 3,04 mmol ou 1 eq.), le monomère B7 (2,196 g soit 3,04 mmol ou 1 eq.), le carbonate de sodium anhydre (0,968 g, 9,13 mmol, 3 eq.), du N,N-diméthylacétamide anhydre (20 ml) et du toluène (4,0 ml, agent azéotropique). Le ballon de réaction est chauffé à 100°C dans un bain d'huile pendant deux heures (distillation azéotropique). La vanne de circulation du toluène est ensuite fermée et le toluène est distillé à 100°C. La température du bain d'huile est ensuite augmentée à 148°C et les résidus de toluène sont éliminés par distillation pendant 1 heure supplémentaire de façon à ce que tout le toluène soit retiré de la réaction et que la température augmente à 140°C à l'intérieur du ballon. Le toluène est vidangé du séparateur "Dean Stark" et la température du bain d'huile est augmentée à 159°C, puis maintenue à cette valeur toute la nuit.

Après 20 h environ, on retire le ballon du bain d'huile et on laisse refroidir jusqu'à 130°C environ à l'intérieur du ballon de réaction. 8 mg de 4-fluorobenzophénone sont alors dissous dans 5 ml de N,N-diméthylacétamide anhydre, le tout ajouté à la réaction à l'aide d'une seringue. Le ballon est replacé dans le bain d'huile et la réaction continue à environ 145°C (environ 158°C dans le bain d'huile) pour 4 h supplémentaires. On laisse refroidir le mélange réactionnel à température ambiante ; le produit obtenu est ensuite versé dans 200 ml dé 2-propanol (isopropanol). Le précipité fibreux est récupéré par filtration.

Le polymère est alors séché sous vide à 80°C pendant toute la nuit. Le carbonate de sodium est extrait du polymère en plongeant ce dernier dans 50 ml d'eau distillée sous agitation avec un barreau magnétique pendant 30 min. Le pH de la solution est ajusté jusqu'à 7 par ajout goutte à goutte de HCl (aq) à 10%. Le polymère est ensuite séché à 80°C sous vide (environ 12 heures).

La formule du Polymère 7 ainsi obtenu, sous forme sulfoné et bloqué benzophénone, est représentée à la figure 22, ainsi que son spectre RMN ¹H (500 MHz), dissous dans DMSO-*d₆*.

### V-6. Synthèse du Polymère 8

Cet exemple décrit de manière détaillée la synthèse du Polymère 8 selon un procédé déjà commenté à la figure 14, à partir du Monomère A8 (ou Composé 2 conforme à l'invention) et du Monomère B8 (ou Composé 9 non conforme à l'invention) précédemment décrits, ce Polymère 8 étant obtenu ici d'une part sous forme sulfoné et d'autre part bloqué par des groupements benzophénone.

Le monomère B8 et Na₂CO₃; sont tout d'abord séchés à 150°C toute la nuit (sous vide), séparément, puis ils sont mélangés ensemble pendant 1 h à 160°C. Le Monomère A8 est lui aussi séché à 80°C (sous vide) toute la nuit.

La polymérisation est conduite dans un ballon tricol de 100mL. Le ballon est surmonté d'une entrée d'azote, d'un thermomètre, d'un agitateur et d'un séparateur "Dean Stark" surmonté d'un réfrigérant. Les parties en verre de l'appareillage (incluant le réfrigérant et le séparateur "Dean Stark") sont séchés sous vide à l'aide d'un pistolet à air chaud. Le ballon est chargé avec le Monomère A8 (0,848 g soit 1,52 mmol), le Monomère B8 (1,22 g soit 1,52 mmol), le carbonate de sodium anhydre (0,48 g soit 4,57 mmol ; excès de trois fois), le N,N-diméthylacétamide sec, le DMA (20 ml) et le toluène (4 ml ; agent azéotropique). Le ballon de réaction est chauffé dans un bain d'huile à 100°C. La température du bain d'huile est ensuite augmentée à environ 148°C et le toluène résiduel est distillé (140°C à l'intérieur du ballon de réaction).

La trappe du séparateur "Dean Stark" est vidée (toluène vidangé) et la température du bain d'huile est augmentée à environ 159°C (environ 150°C à l'intérieur du ballon), puis maintenue à cette température durant environ 20 heures.

Puis, la température de la réaction est abaissée à 100°C à l'intérieur (le ballon est monté au-dessus du bain d'huile) puis 4 mg de 4-fluorobenzophénone dissous dans 5 ml de DMA sont injectés dans la réaction à l'aide d'une seringue. La réaction de blocage est ensuite poursuivie dans un bain d'huile réglé à environ 145°C (température interne) pendant 4 h. On laisse le mélange réactionnel refroidir à température ambiante, puis le polymère est versé dans 300 ml d'isopropanol. Le précipité fibreux est récupéré par filtration et séché au four à 80°C toute la nuit (sous vide). Le carbonate de sodium est retiré du polymère par lavage dans 30 ml d'eau et acidifié par addition goutte à goutte de HCl 10% jusqu'à pH 7. Le polymère final ainsi obtenu est séché à 100°C sous vide.

L'analyse RMN donne les résultats suivants :
*¹H NMR (500 MHz) DMSO-d₆ : 7.08-7.09 (d, 2H), 7.17-719 (d, 4H), 7.39-7.40 (d, 4H), 7.62-7.64 (m, 6H), 7.80-7.82 (d, 2H), 7.79-7.85 (m, 8H), 8.27-8.28 (s, 2H), 8.65-8.66 (d, 4H), ), 8.69-8.70 (d, 2H).*

### V-7. Fabrication de membranes PEM

Dans cet essai, des membranes de Polymère 1, Polymère 2 et Polymère 8 sont préparées selon la technique dite de *"solvent casting"* comme décrit ci-après.

Le polymère (625 mg) préalablement dissous dans 8 ml de N,N-diméthylacétamide est filtré à travers un microfiltre (société "Millipore") en PTFE (polytétrafluoroéthylène) ayant une taille de pores de 0,45 µm environ. Puis la solution de polymère ainsi filtrée est coulée dans un moule constitué de deux plaques de verre superposées, la plaque supérieure comportant un évidement (dimensions 9 cm x 9cm) de profondeur égale à 1 mm ; elle est ensuite chauffée à 50°C pendant 24 h, puis 2 h à 60°C. Puis les traces de solvant organique sont évacuées de la membrane ainsi formée en plongeant cette dernière dans un bain d'eau distillée pendant environ 12 h.

Après séchage final à 60°C pendant 2 h sous vide, une membrane robuste et transparente, d'épaisseur égale à environ 50 µm, est ainsi obtenue, prête pour caractérisation.

### V-8. Caractérisation des membranes PEM

### V-8-A) Conductivité protonique

Pour l'acidification de la membrane (pour rappel, échange du cation M⁺ par H⁺), les Polymères 1, 2 et 8 sont initialement plongés dans 200 ml de H₂SO₄ (aq.) respectivement 3,8 M (pour le Polymère 1) et 1,9 M (pour les Polymères 2 et 8), pendant 2 h. On utilise de l'acide H₂SO₄ distillé deux fois (Sigma Aldrich) pour éviter les traces de métaux. De l'eau distillée est ajoutée ensuite en plusieurs étapes (durée totale environ 12 h) pour atteindre un pH égal à 7 ; la membrane est ensuite conservée ainsi dans l'eau distillée pendant toute la nuit (environ 12 heures).

La conductivité protonique de la membrane exprimée en S/cm (Siemens par centimètre) est déterminée comme indiqué ci-après.

Des membranes en forme de disques de 2 cm de diamètre (épaisseur 50 µm) sont découpées en utilisant un emporte-pièce. La conductivité protonique de la membrane est déterminée par la mesure de la partie réelle (Ohmique) et de la partie imaginaire (Capacitance) de l'impédance complexe, ceci dans la plage de fréquences se situant entre 100 kHz et 10 Hz (avec amplitude de 100 mV AC). Les mesures sont faites avec un potentiostat à impédance/AC (Zahner, Allemagne). Des graphes de Nyquist sont générés par les mesures d'un empilement successif de une, deux, trois et jusqu'à six membranes (totalement humidifiées) prises en sandwich entre deux électrodes en platine de même forme circulaire que les membranes.

Pour chaque mesure, on reporte la valeur interceptant l'axe réel de graphe de Nyquist, c'est-à-dire une valeur de la composante imaginaire de l'impédance à zéro. En général ces points sont alignés sur une droite affine dont la pente détermine directement la valeur de la résistance de la membrane. Son ordonnée à l'origine détermine la résistance de contact entre les membranes et les électrodes en platine. Ces dernières valeurs et la connaissance de l'épaisseur permettent de calculer de manière connue la résistivité de la membrane ; l'inverse de cette valeur est la conductivité.

Ainsi testées, les membranes issues des Polymère 1, Polymère 2 et Polymère 8 ont montré des valeurs de conductivité protonique remarquables, respectivement égales à 89 mS/cm, 73 mS/cm et 35 mS/cm à 25°C (humidité 100%), du même ordre de grandeur que, voire même supérieure à la valeur de conductivité (environ 70 mS/cm) mesurée sur la membrane commerciale ("Nafion^{®} 112") de même épaisseur et testée rigoureusement dans les mêmes conditions.

### V-8-B) Capacité d'absorption d'eau et stabilité dimensionnelle

Une fois la membrane acidifiée, elle est séchée sous vide à 100°C pendant 2 heures. Son poids est immédiatement mesuré avant qu'elle ne capte l'humidité de l'air. Puis les échantillons de membrane sont immergés dans de l'eau distillée à température ambiante jusqu'à saturation (à ce stade, aucune prise de poids supplémentaire due à l'eau n'est alors observée).

La capacité d'absorption de l'eau, exprimée en %, est calculée comme la différence entre les poids de la membrane humide et de la membrane sèche. La stabilité dimensionnelle, également exprimée en %, est le rapport entre la dimension principale de la membrane sèche et la dimension principale de la membrane totalement humidifiée.

On note que les membranes des Polymère 1, Polymère 2 et Polymère 8 ont une capacité d'absorption d'eau respectivement égale à 27%, 17% et 20% de leur poids, comparativement à une valeur d'environ 23% pour la membrane commerciale ("Nafion^{®} 112"). Leur stabilité dimensionnelle est respectivement égale à 20%, 5% et 1%, comparativement à une valeur de 7% pour la membrane commerciale témoin.

En d'autres termes, on constate que ces membranes issues des monomères conformes à l'invention présentent de manière inattendue non seulement une capacité d'absorption d'eau réduite mais aussi une stabilité dimensionnelle remarquable, autant de facteurs qui sont déterminants pour l'endurance et la stabilité chimique de la membrane en fonctionnement dans une pile à combustible PEM.

### V-8-C) Morphologie de surface

Des coupes horizontales et transversales de membrane sont réalisées (chaque échantillon d'épaisseur environ 70 nm), puis sont enrobées dans une résine époxy liquide. La résine est alors polymérisée à 60°C pendant 48 h en présence d'un durcisseur et d'un accélérateur.

Après imprégnation des échantillons de membrane dans une solution aqueuse d'acétate d'uranyle (UO²⁺ CH3COO⁻) puis de citrate de plomb, la morphologie de la membrane est observée en utilisant un microscope électronique à transmission (Philips TEM CM100 ; grossissement 245 000).

Sur la figure 23 sont reproduits les clichés de microscopie électronique enregistrés respectivement sur une coupe horizontale (Fig. 23A) et sur une coupe transversale (Fig. 23B) d'une membrane conforme à l'invention (Polymère 1).

Une taille de pores moyenne égale à 2,4 nm avec un écart type de 0,5 nm constitue un résultat particulièrement remarquable et inattendu pour l'homme du métier. Comparativement aux membranes commerciales connues, l'invention permet donc l'obtention d'une morphologie de surface fortement améliorée, avec d'une part des tailles de pores très sensiblement réduites, d'autre part une distribution particulièrement étroite des tailles ; de telles caractéristiques sont déterminantes pour la performance électrique globale de la membrane, pour ses propriétés d'imperméabilité aux gaz et son endurance finale.

### V-8-D) Performance dans une pile à combustible PEM

Les performances des membranes peuvent être testées sur un banc de test pour piles à combustible sur lequel la température, la pression, le débit et l'humidité des gaz peuvent être réglés. Les gaz utilisés sont de l'hydrogène et de l'oxygène purs, à une température de 65°C.

La pile utilisée dans ces essais est constituée d'une seule cellule comportant la membrane de polymère à tester, disposée entre deux couches "GDE" (*Gas-Diffusion Electrode*), deux plaques bipolaires en graphite et deux électrodes standard ("ELE 0107" de Johnson Matthey) ayant une teneur en platine d'environ 0,4 mg/cm².

La membrane à tester est tout d'abord séchée entre deux non-tissés (qualité chambre stérile, "Sontara Micropure 100" - fournisseur DuPont). Elle est ensuite pressée entre deux plaques de verre, à 60°C pendant 3 h. L'assemblage MEA est obtenu par pressage à chaud d'une couche de catalyse de Pt/C disposée de chaque côté de la membrane (115°C, 125 MPa). A ce stade l'assemblage MEA peut être assemblé entre deux plaques bipolaires pour former une cellule de pile à combustible prête à fonctionner lorsque elle est alimentée en hydrogène et oxygène.

Pour les besoins du test, la pile est soumise à des conditions stationnaires (0,7 V) ou à des situations d'arrêt et démarrage ou "OCV" (*Open Circuit Voltage),* afin de manière connue de soumettre la membrane aux conditions de fonctionnement les plus agressives (e.g. peroxydes, radicaux libres, etc.) et d'en déduire finalement sa résistance chimique globale.

La figure 24 reproduit la courbe dite de polarisation, la tension de cellule étant enregistrée en fonction de la densité de courant délivrée par la pile, d'une part pour la membrane constituée par le Polymère 1 (courbe C_{A}), d'autre part pour la membrane commerciale (polymère "Nafion^{®} 112", courbe C_{B}).

La lecture de ces deux courbes conduit aux commentaires suivants ;
- tout d'abord, à haute tension et courant nul (circuit électrique ouvert), on note que la tension de polarisation est équivalente pour les deux membranes, ce qui illustre pour l'homme du métier une perméabilité aux gaz (O₂ et H₂) équivalente ;
- ensuite, on observe une pente sensiblement identique des deux courbes dans leur partie linéaire centrale (typiquement entre 200 et 1200 mA/cm²), ce qui témoigne d'une performance électrique identique des deux membranes, sans même une optimisation particulière des électrodes (anode et cathode) pour la membrane spécifique de l'invention ;
- enfin, à haut courant et basse tension (typiquement au-delà de 1200 mA/cm²), on observe que le comportement des deux membranes reste similaire, ce qui confirme une très bonne conductibilité protonique de la membrane à courant élevé.

En conclusion, les monomères de l'invention permettent de fabriquer des polymères et membranes PEM qui, de manière inattendue, présentent une stabilité chimique et dimensionnelle, une conductivité ionique au moins équivalentes sinon supérieures à celles des membranes commerciales du type Nafion® développées pourtant depuis très longtemps ; ces polymères présentent en outre une stabilité chimique et une résistance à l'oxydation remarquables, en comparaison avec les polymères triazine de l'art antérieur.

En outre, comparés aux monomères triazine de l'art antérieur, les monomères de l'invention sont relativement peu coûteux et sont susceptibles d'être mis en oeuvre selon des procédés de synthèse simples, économiques et respectueux de l'environnement.

## Revendications

1. Monomère triazine soufré répondant à la formule (I) : 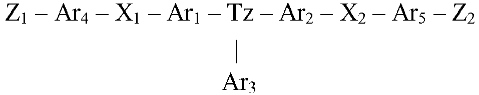 dans laquelle:
- le symbole Tz représente le noyau triazine-1,3,5 ;
- les symboles X₁ et X₂, identiques ou différents, représentent S, SO ou SO₂ ;
- les symboles Ar₁, Ar₂, Ar₄ et Ar₅, identiques ou différents, représentent un groupe phénylène, substitué ou non substitué ;
- le symbole Ar₃ représente un groupe phényle, substitué ou non substitué ;
- les symboles Z₁ et Z₂, identiques ou différents, sont choisis dans le groupe constitué par les halogènes, hydroxyle, alcoxyles, thiol, carboxyles, carboxylates, amino, sulfonamido, chlorure d'acyle, chlorure de sulfonyle, fluorure de sulfonyle, et isocyanate.

2. Monomère triazine selon la revendication 1, dans lequel Z₁ et Z₂, identiques ou différents, sont choisis dans le groupe constitué par les halogènes, hydroxyle et thiol.

3. Monomère triazine selon la revendication 2, répondant à la formule :

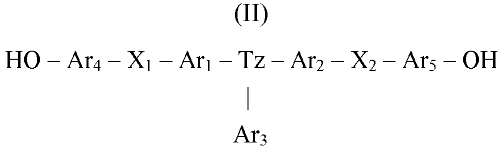

4. Monomère triazine selon la revendication 2, dans lequel Z₁ et Z₂, identiques ou différents, sont des halogènes.

5. Monomère triazine selon la revendication 4, répondant à la formule :

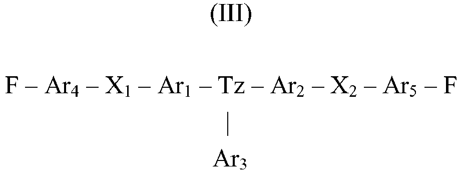

6. Monomère triazine selon la revendication 4, répondant à la formule :

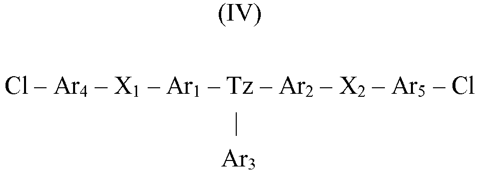

7. Monomère triazine selon l'une quelconque des revendications 1 à 6, ledit monomère étant un monomère sulfoné porteur d'au moins un groupe sulfonique -SO₃H ou un groupe sulfonate -SO₃M (M représentant un cation de métal alcalin).

8. Monomère triazine selon la revendication 7, dans lequel le groupe sulfonique ou sulfonate est porté par au moins un groupe phényle ou phénylène ou, le cas échéant, par au moins un substituant des groupes phényle ou phénylène.

9. Procédé de synthèse d'un polymère triazine par polycondensation d'au moins un monomère triazine selon l'une quelconque des revendications précédentes.

10. Utilisation d'un monomère triazine selon l'une quelconque des revendications précédentes, pour la fabrication d'une membrane de polymère utilisable dans une pile à combustible du type PEM.

## Patentansprüche

1. Schwefelhaltiges Triazin-Monomer der Formel (I): 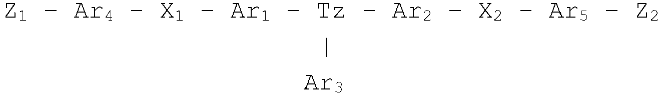 wobei:
- das Symbol Tz für den 1,3,5-Triazinkern steht;
- die Symbole X₁ und X₂ gleich oder verschieden sind und für S, SO oder SO₂ stehen;
- die Symbole Ar₁, Ar₂, Ar₄ und Ar₅ gleich oder verschieden sind und für eine substituierte oder unsubstituierte Phenylengruppe stehen;
- das Symbol Ar₃ für eine substituierte oder unsubstituierte Phenylgruppe steht;
- die Symbole Z₁ und Z₂ gleich oder verschieden sind und aus der Gruppe bestehend aus Halogenen, Hydroxyl, Alkoxygruppen, Thiol, Carboxylgruppen, Carboxylatgruppen, Amino, Sulfonamido, Acylchlorid, Sulfonylchlorid, Sulfonylfluorid und Isocyanat ausgewählt sind.

2. Triazin-Monomer nach Anspruch 1, wobei Z₁ und Z₂ gleich oder verschieden sind und aus der Gruppe bestehend aus Halogenen, Hydroxyl und Thiol ausgewählt sind.

3. Triazin-Monomer nach Anspruch 2 der Formel:

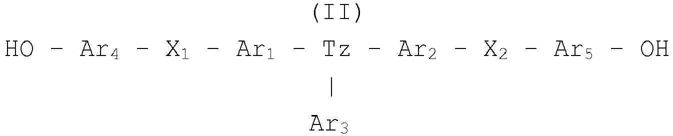

4. Triazin-Monomer nach Anspruch 2, wobei Z₁ und Z₂ gleich oder verschieden sind und Halogene sind.

5. Triazin-Monomer nach Anspruch 4 der Formel:

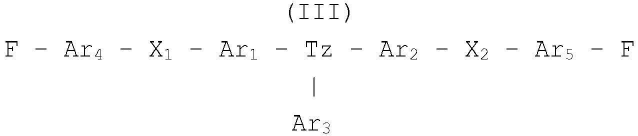

6. Triazin-Monomer nach Anspruch 4 der Formel:

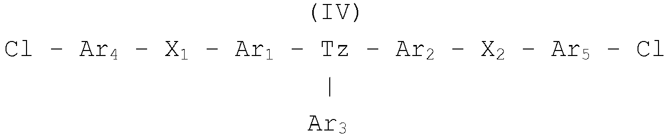

7. Triazin-Monomer nach einem der Ansprüche 1 bis 6, wobei es sich bei dem Monomer um ein sulfoniertes Monomer mit mindestens einer Sulfonsäuregruppe -SO₃H oder einer Sulfonatgruppe -SO₃M (wobei M für ein Alkalimetallkation steht) handelt.

8. Triazin-Monomer nach Anspruch 7, wobei die Sulfonsäure- oder Sulfonatgruppe an mindestens eine Phenyl- oder Phenylengruppe oder gegebenen-falls an mindestens einen Substituenten der Phenyl- oder Phenylengruppen gebunden ist.

9. Verfahren zur Synthese eines Triazin-Polymers durch Polykondensation mindestens eines Triazin-Monomers nach einem der vorhergehenden Ansprüche.

10. Verwendung eines Triazin-Monomers nach einem der vorhergehenden Ansprüche zur Herstellung einer Polymermembran, die in einer Brennstoffzelle vom PEM-Typ verwendet werden kann.

## Claims

1. Sulphur-containing triazine monomer corresponding to the formula (I): 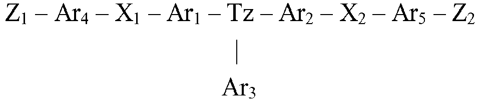 in which:
- the symbol Tz represents the 1,3,5-triazine nucleus;
- the symbols X₁ and X₂, which are identical or different, represent S, SO or SO₂;
- the symbols Ar₁, Ar₂, Ar₄ and Ar₅, which are identical or different, represent a substituted or unsubstituted phenylene group;
- the symbol Ar₃ represents a substituted or unsubstituted phenyl group;
- the symbols Z₁ and Z₂, which are identical or different, are selected from the group consisting of halogens, hydroxyl, alkoxyls, thiol, carboxyls, carboxylates, amino, sulphonamido, acyl chloride, sulphonyl chloride, sulphonyl fluoride and isocyanate.

2. Triazine monomer according to Claim 1, in which Z₁ and Z₂, which are identical or different, are selected from the group consisting of halogens, hydroxyl, thiol.

3. Triazine monomer according to Claim 2, corresponding to the formula:

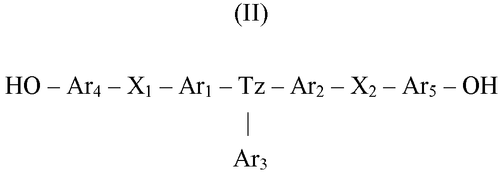

4. Triazine monomer according to Claim 3, in which Z₁ and Z₂, which are identical or different, are halogens.

5. Triazine monomer according to Claim 4, corresponding to the formula:

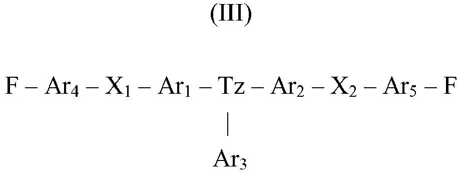

6. Triazine monomer according to Claim 4, corresponding to the formula:

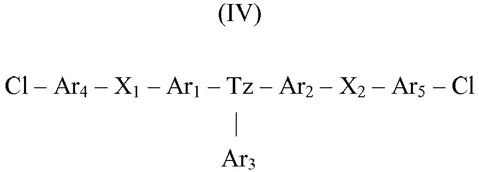

7. Triazine monomer according to any one of Claims 1 to 6, the said monomer being a sulphonated monomer bearing at least one sulphonic group -SO₃H or one sulphonate group - SO₃M (M representing an alkali metal cation).

8. Triazine monomer according to Claim 7, in which the sulphonic or sulphonate group is borne by at least one phenyl or phenylene group or, if appropriate, by at least one substituent of the phenyl or phenylene groups.

9. Process for the synthesis of a triazine polymer by polycondensation of at least one triazine monomer according to any one of the preceding claims.

10. Use of a triazine monomer according to any one of the preceding claims for the manufacture of a polymer membrane which can be used in a fuel cell of the PEM type.
